# EUROPEAN PATENT APPLICATION

(11) **EP 4 759 307 A1**
(43) Date of publication of application: **17.06.2026**
(21) Application number: 24219628.5
(22) Date of filing: 12.12.2024
(51) Int. Cl.: A61K 31/122, A61K 31/192, A61P 3/00, A23L 5/00, A61P 43/00

(54) **4-HYDROXYBENZOIC ACID FOR TREATING COENZYME Q10 DEFICIENCY**

(71) Applicant: Heinrich-Heine-Universität Düsseldorf Körperschaft des Öffentlichen Rechts, 40225 Düsseldorf (DE)
(72) Inventor: Distelmaier, Felix, 40225 Düsseldorf (DE)
(74) Representative: Heide, Anna Katharina

(57) **Abstract**

Provided are 4-hydroxybenzoic acid (4-HBA) as well as analogue and precursor thereof and a composition comprising 4-hydroxybenzoic acid (4-HBA), or an analogue or precursor thereof, respectively, for use in the treatment of coenzyme Q₁₀ (CoQ₁₀) deficiency in human patients. Further provided are methods for screening, identifying, and obtaining a drug suitable for the treatment of CoQ₁₀ deficiency using iPSC-based tissue models.

## Description

### FIELD OF THE INVENTION

The present invention relates to 4-hydroxybenzoic acid (4-HBA), or an analogue or precursor thereof or a composition comprising 4-hydroxybenzoic acid (4-HBA), or an analogue or precursor thereof, respectively, for use in the treatment of coenzyme Q₁₀ (CoQ₁₀) deficiency in human patients. The present invention further relates to methods for screening, identifying, and obtaining of a drug suitable for the treatment of CoQ₁₀ deficiency using iPSC-based tissue models as well as to iPSC-based tissue models mimicking CoQ₁₀ deficiency.

### BACKGROUND OF THE INVENTION

The endogenous synthesis of coenzyme Q₁₀ (CoQ₁₀) is a metabolic pathway that is essential for cellular energy homeostasis. Numerous enzymes participate in this pathway and pathogenic variants in associated genes may cause human disease. These primary CoQ₁₀ deficiency disorders comprise a broad spectrum of clinical manifestations, ranging from neonatal lactic acidosis with multiple organ involvement, encephalopathy, seizures, muscular hypotonia and early death over later-onset neurological features including ataxia, sensorineural hearing loss, exercise intolerance, seizures, optic atrophy, autonomic dysfunction, and stroke-like episodes up to subacute neurological deterioration in late adulthood. Further potential organ manifestations are hypertrophic cardiomyopathy as well as steroid-resistant nephrotic syndrome. In general, disease-onset and clinical features of patients with CoQ₁₀ deficiency are highly heterogeneous (for overview see Salviati *et al.,* in: Pagon et al., (Eds.), GeneReviews®, University of Washington, Seattle, Seattle (WA), 1993-2017).

The standard treatment of CoQ₁₀ deficiency disorders is oral CoQ₁₀ administration. However, in the majority of affected individuals the treatment effect is unsatisfactory. The reason for this poor treatment response has multiple causes including low bioavailability and insufficient uptake into the central nervous system. Moreover, it is unclear whether externally supplemented CoQ₁₀ sufficiently reaches the mitochondrial respiratory chain, where it participates in ATP production or if it mainly accumulates in lipid membranes due to its chemical structure and properties. Accordingly, novel treatment strategies are needed for addressing CoQ₁₀ deficiency.

This technical problem is solved by the embodiments characterized in the claims and described further below and illustrated in the Examples and Figures.

### SUMMARY OF THE INVENTION

The present invention relates to 4-hydroxybenzoic acid (4-HBA), or an analogue or precursor thereof for use in the treatment of coenzyme Q10 (CoQ10) deficiency in human patients. The present invention is based on the surprising finding that administration of 4-hydroxybenzoic acid (4-HBA) to a child who was diagnosed with COQ2-associated primary disorder of coenzyme Q₁₀ (CoQ₁₀) biosynthesis and who showed developmental delay and muscular hypotonia, speech delay, chronic lactic acidosis, recurrent vomiting and failure to thrive, generalized edema and a nephrotic syndrome as well as proteinuria and small bilateral lesions in the thalami and a lactate peak in the white matter, led to a remarkable improvement of the metabolic parameters and clinical manifestations of the CoQ₁₀ deficiency. In particular, as shown in Example 1, already after one month of treatment with 4-HBA, clear reduction of proteinuria was noted (normalization of albumin in urine already after one month of treatment with 4-HBA) and metabolic parameters improved as well (lactate levels around 2-3 mmol/l). Clinically, the parents, physiotherapists and pediatricians noted a drastic improvement in terms of muscle strengths, mobility, exercise tolerance and fine motor function. Already after 2.5 months of treatment with 4-HBA, the toddler was able to walk unsupported for short distances, which was not possible prior to treatment. Also, the eating behavior improved, and recurrent vomiting stopped completely. Accordingly, the toddler significantly gained weight (1.1 kg in 2 ½ months). Apart from motoric functions, alertness and speech improved with learning of several new words. Surprisingly, also the social behavior changed. Prior to treatment, the toddler was extremely anxious and did not play age-appropriately. This changed dramatically with much better interaction and participation in supportive treatments. The study also proves that administration of 4-HBA has a superior treatment effect in comparison to administration of CoQ₁₀. After the child had been diagnosed with CoQ₁₀ deficiency, an oral supplementation therapy with CoQ₁₀ was started. Over the course of three months, some improvement in terms of better exercise tolerance were noted (child was more active and having less episodes with vomiting and apathy) but no major clinical changes have occurred. In contrast, already after 2.5 months of treatment with orally administered 4-HBA, major metabolic and clinical changes were noted which proves the superiority of the 4-HBA therapy over CoQ₁₀-therapy. Furthermore, no toxicity or side effects were observed during the treatment period. Accordingly, it was shown for the first time that administration of 4-HBA to a patient does not only treat CoQ₁₀ deficiency but is also safe and thus, particularly useful for treatment of children.

Thus, the present invention preferably relates to 4-HBA for use in the treatment of a CoQ₁₀ deficiency which is associated with a pathogenic variant of the COQ2 enzyme (4-HBA-polyprenyltransferase), i.e., with a COQ2-associated CoQ₁₀ deficiency leading to a defective CoQ₁₀ biosynthesis pathway.

Previously, *in vitro* studies have shown that supplementation of 4-HBA restores endogenous CoQ₁₀-biosynthesis in COQ2-deficient cell lines; see Herebian et al., Ann Clin Transl Neurol 4 (2017), 902-908. However, regarding the therapeutic potential of 4-HBA the authors of Herebian *et al.,* 2017, noted that the exact function of COQ2 is still not fully understood and that the importance of *in vivo* studies to establish a drug profile analysis for 4-HBA and to evaluate its therapeutic potential under clinical conditions. Accordingly, though a mouse model had been developed for studying the effect of 4-HBA on COQ2 defects (Corral-Sarasa et al., Cell Reports 43 (2014), 114148), which provided some scientifically interesting results, the authors of Corral-Sarasa *et al.,* 2024, acknowledge the limitations of the study and that it is not possible to contemplate the potential translation of the mouse therapy model into clinical applications.

At the time the invention was made, it remained unclear whether 4-HBA and related compounds could restore CoQ₁₀ deficiency and, if so, to what extent clinical symptoms could be ameliorated. Furthermore, even if 4-HBA could theoretically restore CoQ₁₀ levels, it was uncertain whether treatment with a therapeutically effective amount of 4-HBA would be safe and free from harmful side effects. Despite these uncertainties when it comes to children regarding the potential benefits and risks of the treatment, the inventors decided to embark on a clinical study, which, fortunately for the patients, led to the pioneering discovery that the administration of 4-HBA indeed ameliorates and heals the symptoms of CoQ₁₀ deficiency in a patient.

Furthermore, during the case study performed within the scope of the present invention, it has been shown for the first time that a patient after the onset of the disease can be successfully treated with 4-HBA. Accordingly, with the results of the present study, it has been shown for the first time that 4-HBA can be used in the treatment of patients being affected by symptoms of CoQ₁₀ deficiency.

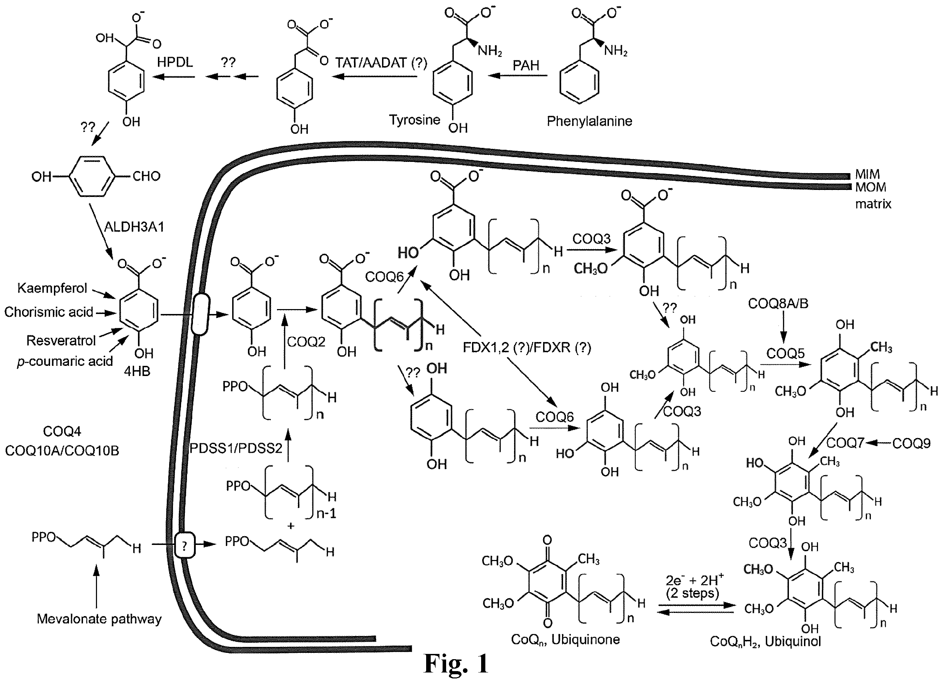

CoQ₁₀ is a cofactor required for normal functioning of the mitochondrial respiratory chain and 4-HBA is the precursor of the benzoquinone ring of CoQ₁₀. 4-HBA is first prenylated by COQ2 and seven reactions produce the fully substituted benzoquinone ring of CoQ₁₀. The CoQ₁₀ biosynthesis pathway is depicted for example in Fig. 1 of Staiano et al., Antioxidants 12 (2023), 1469 and reproduced in simplified form in present Fig. 1. In previous *in vitro* studies it has been shown that clinically relevant COQ2 mutations are associated with a residual enzyme activity that can be stimulated by increasing substrate availability (Herebian *et al.,* 2017). In particular, it was found that the COQ2 enzyme most likely contains a substrate transportation pathway, which shuttles 4-HBA trough the enzyme to the catalytic site. Mapping of known disease-causing *COQ2* mutations on an *in silico* model showed, that these mutations cause changes alongside the transport pathway, most likely leading to hindered transport. Accordingly, increasing the dose of 4-HBA seems to overcome this obstacle and thereby leads to a reactivation of COQ2 activity. Thus, in one embodiment, the COQ2 enzyme has residual enzyme activity and the 4-HBA supplementation functions as substrate-enhancement therapy in the treatment of COQ2-associated CoQ₁₀ deficiency.

The mutations present in the COQ2 enzyme leading to the disease phenotype are not limited as long as the enzyme has residual activity and activity that can be restored after 4-HBA supplementation, respectively, *i.e.,* the patient population amendable to the treatment in accordance with the present invention is not limited to a particular pathogenic form of COQ2 as long as the enzyme has residual activity and activity that can be restored after 4-HBA supplementation, respectively. The child which has been subject of the case study of the present invention has compound-heterozygous variants in the COQ2 gene, wherein at position 571 of the coding sequence, the guanine (G) is changed to cytosine (C) (c.571G>C) and at position 692 of the coding sequence, the guanine (G) is changed to thymine (T) (c.692G>T), and/or wherein at position 191 in the protein, the valine (Val) is substituted with leucine (Leu) (p.Val191Leu) and at position 231 in the protein, the serine (Ser) is substituted with isoleucine (Ile) (p.Ser231Ile). Accordingly, in one embodiment, the human subject to be treated in accordance with the present invention has compound-heterozygous variants in the COQ2 gene, preferably c.571G>C, p.Val191Leu; c.692G>T, p.Ser231Ile. In another embodiment, the subject to be treated is compound homozygous for the variants in the COQ2 gene.

It is prudent to expect that also treatment or supplementation with a 4-HBA precursor is efficient in the treatment of COQ2-associated CoQ₁₀ deficiency. For example, as visualized in Fig. 1, phenylalanine, tyrosine, kaempferol, chorismic acid, resveratrol, p-coumaric acid, and also para-aminobenzoic acid (pABA) are precursors of 4-HBA. Furthermore, in Herebian et al., Ann Clin Transl Neurol. 4 (2017), 902-908, it has been shown that next to 4-HBA, also its precursor compounds 4-hydroxyphenylpyruvic acid (4-HPPA), 4-hydroxybenzaldehyde (4-HBAL), and L-tyrosine (L-Tyr) rescued the biochemical defect in three COQ2-deficient fibroblast lines. Thus, in one embodiment, the present invention does not only relate to 4-HBA, but also to one or more of its precursors, preferably to any one of phenylalanine, tyrosine, kaempferol, chorismic acid, resveratrol, p-coumaric acid, pABA, 4-hydroxyphenylpyruvic acid (4-HPPA), and 4-hydroxybenzaldehyde (4-HBAL), more preferably to 4-hydroxyphenylpyruvic acid (4-HPPA), 4-hydroxybenzaldehyde (4-HBAL), and tyrosine, for use in the treatment of COQ2-associated CoQ₁₀ deficiency.

With the surprising finding that 4-HBA treatment is indeed effective in a patient having COQ2-associated CoQ₁₀ deficiency and the knowledge derived therefrom, it is prudent to expect that a CoQ₁₀ deficiency associated with another defective enzyme in the CoQ₁₀ biosynthesis pathway can also be treated with 4-HBA or an analogue, or precursor thereof, either as substrate-enhancement therapy or as bypass therapy. Thus, the present invention relates in general to 4-HBA or an analogue or precursor thereof for use in the treatment of CoQ₁₀ deficiency, also referred to a CoQ₁₀ biosynthesis disorder. In another embodiment a combination of 4-HBA, the analogue and/or precursor thereof or a composition comprising a combination of 4-HBA, the analogue and/or precursor thereof are used in the treatment of CoQ₁₀ deficiency.

In particular, during the last decades, defects in eleven different genes encoding CoQ₁₀ biosynthesis proteins were identified (e.g. PDSS1, PDSS2, COQ2, COQ4, COQ5, COQ6, COQ7, COQ8A/ADCK3, COQ8B/ADCK4, COQ9, and HPDL) and supplementation of cell lines with synthetic CoQ₁₀ precursor compounds demonstrated beneficial effects on CoQ₁₀ biosynthesis protein deficiencies. For example, supplementation with 2,4-dihydroxybenzoic acid showed beneficial effects in COQ7 and COQ9 deficiency and supplementation with vanillic acid showed beneficial effects in COQ9 and COQ6 deficiency (Herebian et al., Molecular Genetics and Metabolism 121 (2017), 216-223, Herebian et al., Ann Clin Transl Neurol 4 (2017), 902-908, Lopez et al., Oxid Med Cell 2019, 3904905).

In general, such bypass strategies may be applicable to other cases of primary CoQ₁₀ deficiency. Accordingly, it is further prudent to expect that a bypass therapy with 4-HBA is also effective in the treatment of HPDL associated CoQ₁₀ deficiency since HPDL is involved in conversion of the precursor tyrosine to 4-HBA (see Fig. 1).

Accordingly, in one embodiment, the CoQ₁₀ deficiency to be treated in accordance with the present invention is COQ1/PDSS1/PDSS2-, HPDL-, COQ2-, COQ4-, COQ5-, COQ6-, COQ7-, COQ8A/ADCK3-, COQ8B/ADCK4-, and/or COQ9-deficiency associated CoQ₁₀ deficiency. In one embodiment, the CoQ₁₀ deficiency to be treated in accordance with the present invention is COQ9 or COQ6 associated CoQ₁₀ deficiency, wherein treatment is preferably performed with the 4-HBA analogue vanillic acid. In one embodiment, the CoQ₁₀ deficiency to be treated in accordance with the present invention is COQ7 or COQ9 deficiency associated with CoQ₁₀ deficiency, wherein treatment is preferably performed with the 4-HBA analogue 2,4-dihydroxybenzoic acid. In one embodiment, the CoQ₁₀ deficiency to be treated in accordance with the present invention is HPDL associated CoQ₁₀ deficiency.

Since successful treatment of a patient after onset of the disease has been shown in the study within the scope of the present invention, wherein the treatment ameliorated the symptoms of CoQ₁₀ deficiency and improved metabolic and clinical parameters in the patient, it is prudent to expect that 4-HBA or the analog or precursor thereof can be used for prevention of CoQ₁₀ deficiency, in particular in prevention of a CoQ₁₀ deficiency phenotype in a subject being at risk of developing a CoQ₁₀ deficiency phenotype. Such a subject amendable for preventive treatment can be a subject which has a genetic predisposition for the disease, for example which has one or more defects in the CoQ₁₀ biosynthesis pathway as described hereinbefore, preferably which has a defect in any one of COQ1/PDSS1/PDSS2, HPDL, COQ2, COQ4, COQ5, COQ6, COQ7, COQ8A/ADCK3, COQ8B/ADCK4, and/or COQ9, or any combination thereof, more preferably which has a defect in COQ2 or HPDL, most preferably in COQ2.

4-HBA and its derivatives are applied as supplements in the food and cosmetic industry (E-substances E 214 and E 215), 4-HBA has a GRAS ("Generally Recognized As Safe") status according to the U.S. Food and Drug Administration (FDA) and oral administration has also been tested in human subjects, wherein its application was reported as safe without any side effects; see for example BUA Stoffberichte 164-166: Kurzberichte; 164 p-Hydroxybenzoesäure, 12/93; 165 Hexabromcyclododecan 08/95; 166 Propylenglykol; 08/95. As mentioned above, in the experiments made within the scope of the present invention, the suitability of 4-HBA as a drug has been shown for the first time and thus, the present invention generally relates to 4-HBA for use as a drug in human patients.

As mentioned above, during the case study performed within the scope of the present invention, it has been shown for the first time that a patient after the onset of the disease can be successfully treated with 4-HBA. In contrast, the COQ2 mouse model developed previously shows perinatal lethality so that the effects of 4-HBA supplementation after disease onset could not be studied. However, this is particularly relevant as CoQ₁₀ deficiency is usually diagnosed in childhood or sometimes even in adults. Accordingly, in one embodiment, the treatment in accordance with the present invention is a postnatal treatment.

CoQ₁₀ deficiency mainly causes three different clinical manifestations: 1) A severe early-infantile form, wherein affected individuals show clinical symptoms shortly after birth with lactic acidosis, kidney dysfunction, cardiomyopathy, epilepsy, and severe encephalopathy. These children have a poor prognosis and usually die within the first months of life. 2) An intermediate phenotype, wherein affected individuals usually manifest with subacute symptoms such as developmental delay, muscular hypotonia, ataxia, epilepsy, and failure to thrive. Kidney function usually deteriorates in the 2^{nd} and 3^{rd} year of life with steroid-resistant nephrotic syndrome. The disease course is progressive and patients may show neurodegeneration with regression and kidney failure with requirement of dialysis. 3) A late onset phenotype in terms of a sub-form of multiple system atrophy, wherein patients show adult-onset dementia, movement abnormalities, visual problems, dysarthria and autonomic dysfunction (constipation, incontinence, etc.).

During the studies made within the scope of the present invention, it was shown that a child could be successfully treated with 4-HBA. Since the cause of the disease, i.e., deficiency of the CoQ₁₀ biosynthesis is the same among all age groups, it is prudent to expect that also adults and newborn can be treated with 4-HBA.

Thus, in one embodiment, the CoQ₁₀ deficiency to be treated in accordance with the present invention is a severe early-infantile form, wherein the affected human subject shows clinical symptoms shortly after birth, preferably wherein disease manifestation occurs within the first 6 months after birth, preferably wherein the one or more clinical symptoms are selected from the group consisting of: lactic acidosis, kidney dysfunction, cardiomyopathy, epilepsy, and severe encephalopathy. Accordingly, in one embodiment, the patient group to be treated in accordance with the present invention comprises neonates, wherein the neonates show at least one or more of the following clinical symptoms: lactic acidosis, kidney dysfunction, cardiomyopathy, epilepsy, and severe encephalopathy. The treatment success may be measured based on an improvement of the metabolic and/or clinical symptoms, for example reduction of lactic acidosis, improvement of kidney function and reversion of encephalopathy. Thus, the term "successfully treated" means that at least one or more of the metabolic and/or clinical symptoms are improved or are ameliorated in a measurable extent.

With regard to this patient group, not only postnatal treatment, in particular after disease onset, is an option, but also prenatal treatment, preferably if the unborn has a genetic predisposition as explained above. In this case, prenatal (preventive) treatment should lead to a symptom-free neonate or to a neonate with only mild symptoms of a CoQ₁₀ deficiency. Accordingly, in one embodiment, the treatment in accordance with the present invention is a prenatal treatment.

In one embodiment, the CoQ₁₀ deficiency to be treated in accordance with the present invention is an intermediate form, wherein the affected human subject shows at least one or more clinical symptoms in early childhood, preferably wherein the one or more clinical symptoms are selected from the group consisting of: developmental delay, muscular hypotonia, ataxia, failure to thrive, epilepsy, kidney deterioration with steroid-resistant nephrotic syndrome, neurodegeneration, retinopathy and optic atrophy. Accordingly, in one embodiment, the patient group to be treated in accordance with the present invention comprises children, preferably in their early childhood, preferably wherein disease manifestation occurs between the age of six months and six years, preferably between the age of six months and three years, preferably wherein the children show at least one or more of the following clinical symptoms: developmental delay, muscular hypotonia, ataxia, failure to thrive, epilepsy, kidney deterioration with steroid-resistant nephrotic syndrome, neurodegeneration, retinopathy and optic atrophy.

In particular, a patient to be treated in accordance with the present invention, which is preferably a child in its early childhood, may have the following phenotype:
(i) proteinuria as measured by the total protein level and albumin level in a urine sample which is above the reference range, preferably wherein the total protein level is > 150 mg/ml and the albumin level is > 30 mg/ml in the urine sample,
(ii) lactic acidosis as measured by the lactate level in a blood sample which is above the standard range, preferably wherein the lactate level in the blood sample is >1.6 mmol/l,
(iii) brain abnormalities including white matter changes, symmetrical lesions of basal ganglia and/or brain stem, cerebellar hypoplasia or atrophy, and a lactate peak detected by brain imaging;
(iv) gastrointestinal symptoms including recurrent vomiting and feeding problems;
(v) reduced muscle strength, mobility, exercise tolerance, motor function, and/or fine motor function compared to peers;
(vi) reduced alertness, poorer speech and/or poorer social behavior compared to peers; and/or
(vii) reduced body weight compared to peers.

As explained in Example 1, treatment in accordance with the present invention resulted in reduction of proteinuria, reduction of lactic acidosis, reduction of vomiting, improvement of muscle strength, improvement of mobility, improvement of exercise tolerance, improvement of motor function, improvement of fine motor function, improvement of alertness, improvement of speech, improvement of social behavior, and gain of weight in the child.

In one embodiment, the CoQ₁₀ deficiency to be treated in accordance with the present invention is a late-onset form, wherein the affected human subject shows one or more clinical symptoms in adult life, preferably wherein the one or more clinical symptoms are selected from the group consisting of: adult-onset dementia, movement abnormalities, visual problems, dysarthria and autonomic dysfunction. Accordingly, in one embodiment, the patient group to be treated in accordance with the present invention comprises adults, preferably between 40 and 70 years of age, wherein the adults show at least one or more of the following clinical symptoms: adult-onset dementia, movement abnormalities, visual problems, dysarthria and autonomic dysfunction.

The treatment success may be measured based on an improvement of the metabolic and clinical symptoms, for example slowing down the progress of dementia and improving memory performance, respectively, improvement of movement and vision as well as improvement of the autonomic function and speech.

During the present study, a dosing strategy was developed which led to a remarkable reduction in disease symptoms in a child. Treatment was started orally at a 4-HBA dose of 3 x 250 mg/day (75 mg/kg body weight/day) and after one week increased to 3 x 500 mg/day (150 mg/kg body weight/day). Accordingly, in one embodiment, 4-HBA is administered at doses which range from 50 mg/kg body weight/day to 250 mg/kg body weight/day, preferably from 50 mg/kg body weight/day to 150 mg/kg body weight/day, preferably from 75 mg/kg body weight/day to 150 mg/kg body weight/day or from 70 mg/kg body weight/day to 140 mg/kg body weight/day. Preferably, the amount of 4-HBA is administered in three separate doses during the day, preferably wherein the doses are administered six to eight hours apart from each other.

In one embodiment, the 4-HBA dose for administration to children ranges from 3 x 150 mg/day to 3 x 850 mg/day, preferably from 3 x 150 mg/day to 3 x 500 mg/day, preferably from 3 x 250 mg/day to 3 x 500 mg/day.

In one embodiment, the 4-HBA dose for administration to adults ranges from about 3 x 250 mg/day to 3 x 6000 mg/day, preferably from 3 x 500 mg/day to 3 x 6000 mg/day, preferably from 3 x 1000 mg/day to 3 x 6000 mg/day, preferably from 3 x 1000 mg/day to 3 x 3500 mg/day, preferably from 3 x 1750 mg/day to 3 x 3500 mg/day.

In one embodiment, the dose is increased over treatment time. In a preferred embodiment, administration is started with a lower dose and within a month, preferably after one week, the dose is increased. In a preferred embodiment, 4-HBA is administered at an initial dose of 50 mg/kg body weight/day to 100 mg/kg body weight/day, preferably at a dose of 75 mg/kg body weight/day or 70 mg/kg body weight/day followed by a subsequent dose of 100 mg/kg body weight/day to 250 mg/kg body weight/day, preferably of 150 mg/kg body weight/day or 140 mg/kg body weight/day within the first month, preferably after the first week of treatment. The first and the subsequent doses are administered daily and preferably in three separate doses as mentioned above. In a preferred embodiment, the initial dose is 75 mg/kg body weight/day and the subsequent dose (until end of treatment) is 150 mg/kg body weight/day. In another preferred embodiment, the initial dose is 70 mg/kg body weight/day and the subsequent dose (until end of treatment) is 140 mg/kg body weight/day. Preferably, the amount of 4-HBA is administered in three separate doses during the day, preferably wherein the doses are administered six to eight hours apart from each other.

In one embodiment, the initial 4-HBA dose for administration to children ranges from 3 x 150 mg/day to 3 x 350 mg/day and is preferably 3 x 250 mg/day, and the subsequent dose ranges from 3 x 350 mg/day to 3 x 850 mg/day and is preferably 3 x 500 mg/day.

In one embodiment, the initial 4-HBA dose for administration to adults ranges from about 3 x 1000 mg/day to 3 x 3500 mg/day and is preferably 3 x 1750 mg/day, and the subsequent dose ranges from 3 x 1750 mg/day to 3 x 6000 mg/day and is preferably 3 x 3500 mg/day.

In a preferred embodiment, the maximal dose is about 3 x 4000 mg/day in adults and about 3 x 550 mg/day in children.

In one embodiment, treatment in accordance with the present invention is lifelong, either at the same dosage or variable dosage dependent on changes in the disease phenotype and/or weight.

4-HBA is an acid and before administration, 4-HBA was dissolved in milk to reduce the acidity. However, especially for older age groups, water is also an appropriate carrier for administration. Accordingly, in a preferred embodiment, 4-HBA is dissolved before administration in milk, milk substitute or breast milk substitute or water.

Currently, the standard treatment of CoQ₁₀ deficiency disorders is oral CoQ₁₀ application. In the present case study, treatment of the child with CoQ₁₀ was stopped before treatment with 4-HBA was initiated. Accordingly, in one embodiment, the treatment in accordance with the present invention comprises administration of 4-HBA or an analogue, or precursor thereof subsequently to administration with CoQ₁₀. Also, simultaneous treatment with 4-HBA or an analogue, or precursor thereof and CoQ₁₀ is encompassed by the present invention. Accordingly, the human subject is treated with 4-HBA or an analogue, or precursor thereof in an effective amount alone or in combination with CoQ₁₀, wherein 4-HBA or an analogue, or precursor thereof is administered concurrently with or subsequently to CoQ₁₀. CoQ₁₀ is administered to the patient in an effective amount and according to already established treatment regimens. A standard dosage for CoQ₁₀ during treatment of CoQ₁₀ deficiency is 30 mg/kg/day. In a preferred embodiment, CoQ₁₀ is orally administered at a dose of about 5 mg/kg body weight per day to about 70 mg/kg body weight per day, preferably about 20 mg/kg body weight per day to about 40 mg/kg body weight per day, and most preferably at a dose of about 30 mg/kg body weight per day.

The present invention also relates to a composition comprising 4-hydroxybenzoic acid (4-HBA), or an analogue or precursor thereof or a composition comprising a combination of 4-HBA, the analogue and/or precursor thereof for use in accordance with the present invention. In the composition, the 4-HBA is the active ingredient and it is either the only active ingredient or another active ingredient can be present, for example, if 4-HBA is used in combination as explained above.

The present invention further relates to a method for identifying and/or obtaining a substance suitable for the amelioration or treatment of CoQ₁₀ deficiency comprising a) contacting neuronal precursor cells (NPCs), differentiated neurons (DNs), or brain organoids with a test substance, wherein the NPCs, DNs and brain organoids have been generated based on iPSCs comprising a pathogenic variant of a protein involved in CoQ₁₀ biosynthesis, and (b) studying the neuronal differentiation in the NPCs, DNs, or brain organoids, wherein an improved neuronal differentiation in comparison to a control is indicative for a substance suitable for the amelioration or treatment of CoQ₁₀ deficiency.

The present invention further relates to a method for identifying and/or obtaining a substance suitable for the amelioration or treatment of CoQ₁₀ deficiency comprising a) contacting kidney precursor cells (KPCs), podocytes, or kidney organoids with a test substance, wherein the KPCs, podocytes, and kidney organoids have been generated based on iPSCs comprising a pathogenic variant of a protein involved in CoQ₁₀ biosynthesis, and (b) studying the tubular and glomerular structure, function, and integrity in the KPCs, podocytes, or kidney organoids, wherein an improved tubular and glomerular structure, function, and integrity in comparison to a control is indicative for a substance suitable for the amelioration or treatment of CoQ₁₀ deficiency.

The present invention further relates to a method for screening a substance for the suitability to ameliorate or treat CoQ₁₀ deficiency comprising (a) contacting neuronal precursor cells (NPCs), differentiated neurons (DNs), or brain organoids with a test substance, wherein the NPCs, DNs and brain organoids have been generated based on iPSCs comprising a pathogenic variant of a protein involved in CoQ₁₀ biosynthesis; (b) studying the neuronal differentiation in the NPCs, DNs, or brain organoids of step (a); and (c) comparing the neuronal differentiation studied in step (b) to that of DNs or brain organoids not subjected to the test substance.

The present invention further relates to a method for screening a substance for the suitability to ameliorate or treat CoQ₁₀ deficiency comprising (a) contacting kidney precursor cells (KPCs), podocytes, or kidney organoids with a test substance, wherein the KPCs, podocytes, and kidney organoids have been generated based on iPSCs comprising a pathogenic variant of a protein involved in CoQ₁₀ biosynthesis; (b) studying the tubular and glomerular structure, function, and integrity in the KPCs, podocytes, or kidney organoids of step (a); and (c) comparing the tubular and glomerular structure, function, and integrity studied in step (b) to that of KPCs, podocytes, or kidney organoids not subjected to the test substance.

In a preferred embodiment, the protein involved in CoQ₁₀ biosynthesis is COQ2 and the CoQ₁₀ deficiency is COQ2 associated CoQ₁₀ deficiency. In another preferred embodiment, the protein involved in CoQ₁₀ biosynthesis is HPDL and the CoQ₁₀ deficiency is HPLD associated CoQ₁₀ deficiency.

In principle, an iPSC with any pathogenic variant of COQ2 (which has residual activity) or HPLC which leads to a defective CoQ₁₀ biosynthesis pathway is suitable for the generation of the precursor cells, differentiated neurons, differentiated podocytes, and organoids used in the methods of the present invention. In a preferred embodiment, the pathogenic variant of COQ2 is c.779G>A, p.Gly260Glu and the pathogenic variant of HPDL is c.779G>A, p.Gly260Glu. The methods of the present invention can in principle be used for the identification and screening of any compound suitable as drug or active substance in general, suitable for the amelioration or treatment of CoQ₁₀ deficiency. In a preferred embodiment, the methods are used for the identification of further 4-HBD derivatives.

The present invention further relates to organoids which comprise iPSCs comprising a pathogenic variant of a protein involved in CoQ₁₀ biosynthesis as starting material. These iPSCs are differentiated into corresponding tissue-specific cell model systems. In one embodiment, the cell model comprises differentiated neurons (DNs) or is a human cerebral/brain organoid, wherein the DNs and human cerebral/brain organoids, in which the cells self-organize into 3D structures resembling the architecture and function of the target organ, have been generated based on iPSCs comprising a pathogenic variant of a protein involved in CoQ10 biosynthesis. In another embodiment, the cell model system comprises podocytes or is a human kidney organoid, wherein the kidney organoid has been generated based on iPSCs comprising a pathogenic variant of a protein involved in CoQ10 biosynthesis. In a preferred embodiment, the protein involved in CoQ₁₀ biosynthesis is COQ2 and the CoQ₁₀ deficiency is COQ2 associated CoQ₁₀ deficiency. In another preferred embodiment, the protein involved in CoQ₁₀ biosynthesis is HPDL and the CoQ₁₀ deficiency is HPLD associated CoQ₁₀ deficiency. Accordingly, the DNs, human cerebral/brain organoids, podocytes, or kidney organoids represent a COQ2 deficiency or HPDL deficiency phenotype.

The iPSC-based cell models can be used in the methods of the present invention and can also be used for assessing CoQ₁₀ levels, mitochondrial respiration, cellular metabolism and distribution, morphology, and/or electrophysiology, as well as tubular and glomerular structure, function, and integrity, respectively.

Further embodiments of the present invention will be apparent from the description and Examples that follow.

### BRIEF DESCRIPTION OF THE DRAWINGS

- **Fig. 1:**: CoQ₁₀ biosynthesis pathway. A compendium of the reactions carried out to synthesize CoQ₁₀ in mammals is depicted including the name of the proteins involved. Proteins whose functions are still unknown are indicated separately. PAH, phenylalanine hydrolase; TAT, tyrosine aminotransferase; AADAT, mitochondrial alpha-aminoadipate aminotransferase; ALDH3A1, aldehyde dehydrogenase 3A1; HPDL, hydroxyphenylpyruvate dioxygenase-like; symbols ? and ?? indicate enzyme/s still not identified. The Figure is prepared based on the publication by Staiano et al., Antioxidants 12 (2023), 1469.

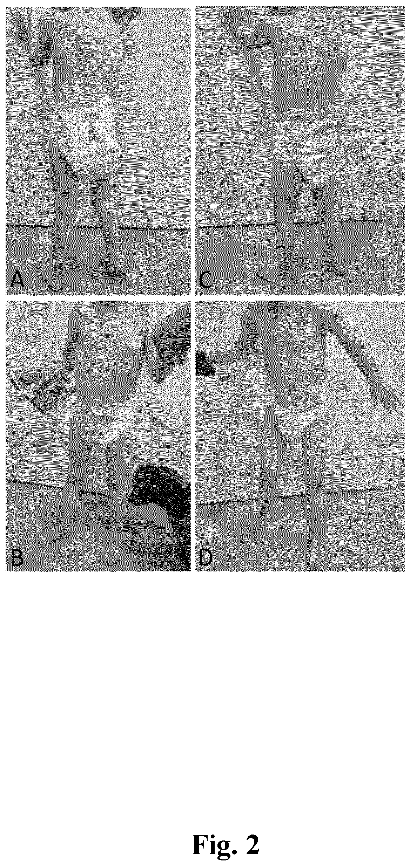

- **Fig. 2:**: Clinical images. A and B, status at the start of 4-HBA treatment. C and D, status after 2 ½ months of 4-HBA treatment. Weight gain and improved muscle relief are visible.

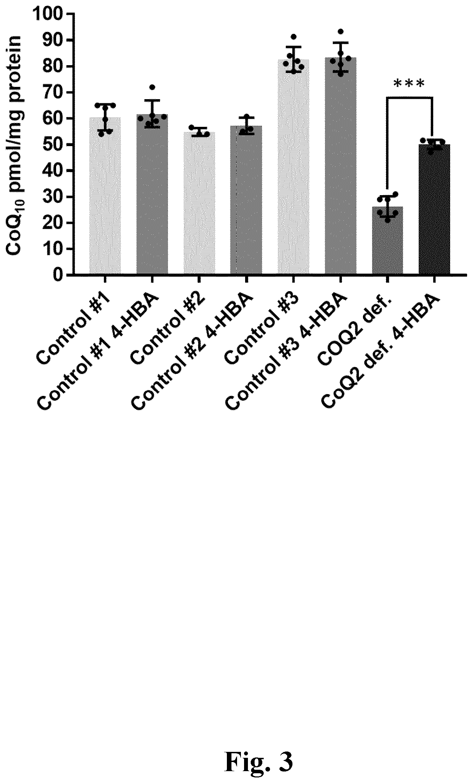

- **Fig. 3:**: Quantification of CoQ₁₀ levels in control and COQ2-deficient fibroblast cell lines measured by UPLC-MS/MS. Bar graphs depict untreated (light color) and treated (dark color) conditions. 4-HBA was used at a concentration of 100 µmol for 14 days. Comparison of the treated patient-derived cells (COQ2 def.) compared to the 4-HBA treated condition (COQ2 def. 4-HBA) showed a highly significant increase in endogenously synthesized CoQ₁₀. Statistics: ***P < 0.001. Statistical significance was assessed using Student's t-test). Experimental data was obtained in three independent experiments.

### DETAILED DESCRIPTION OF THE INVENTION

The present invention relates to 4-hydroxybenzoic acid (4-HBA), or an analogue or precursor thereof as well as to a composition comprising 4-HBA, or an analogue or precursor thereof for use in the treatment or prevention of a coenzyme Q₁₀ (CoQ₁₀) deficiency in a human subject. In another embodiment a combination of 4-HBA, the analogue and/or precursor thereof or a composition comprising a combination of 4-HBA, the analogue and/or precursor thereof are used in the treatment of CoQ₁₀ deficiency. More specifically, the present invention relates to the embodiments as characterized in the claims, disclosed in the description and illustrated in the Examples and Figures further below.

Unless otherwise defined, all technical and scientific terms used herein have the same meaning as commonly understood by one of ordinary skill in the art to which this invention belongs. Although methods and materials similar or equivalent to those described herein can be used in the practice or testing of the present invention, the exemplary methods and materials are described below. All publications, patent applications, patents, and other references mentioned herein are incorporated by reference in their entirety. The materials, methods, and examples are illustrative only and not intended to be limiting. Unless otherwise stated, a term as used herein is given the definition as provided in the Oxford Dictionary of Biochemistry and Molecular Biology, Oxford University Press, 1997, revised 2000 and reprinted 2003, ISBN 0 19 850673 2; Second edition published 2006, ISBN 0-19-852917-1 978-0-19852917-0.

For the avoidance of any doubt it is emphasized that the expressions "in a particular embodiment", "in a further embodiment", "in one embodiment", "in another embodiment" and the like are used and meant such that any of the embodiments described therein are to be read with a mind to combine each of the features of those embodiments and that the disclosure has to be treated in the same way as if the combination of the features of those embodiments would be spelled out in one embodiment. The same is true for any combination of embodiments and features of the appended claims and illustrated in the Examples, which are also intended to be combined with features from corresponding embodiments disclosed in the description, wherein only for the sake of consistency and conciseness the embodiments are characterized by dependencies while in fact each embodiment and combination of features, which could be construed due to the (multiple) dependencies must be seen to be literally disclosed and not considered as a selection among different choices.

The term "between" as used herein includes the endpoints.

As used herein, the term "about," as used herein, refers to a value that is ± 10% of a recited value; preferably ± 5%.

In connection with the present invention, the term "and/or" is understood to mean that all members of a group which are connected by the term "and/or" are disclosed cumulatively in any combination, both alternatively to each other and in each case to each other. This means for the expression "A, B and/or C" that the following disclosure content is to be understood thereunder: a) A or B or C; or b) (A and B); or c) (A and C); or d) (B and C); or e) (A and B and C). For example, in relation to the symptoms of CoQ₁₀ deficiency, "and/or" means that any combination of the symptoms in any mild to severe form may occur simultaneously or consecutively.

As used herein, the terms "treat" or "treatment" refer to both therapeutic treatment and prophylactic or preventative measures, in which the object is to prevent or slow down (lessen) or to decrease to a lower level/extent an undesired physiological change or disorder, such as the development of proteinuria. Beneficial or desired clinical results include, but are not limited to, alleviation of symptoms, diminishment of extent of disease, stabilized (i.e., not worsening) state of disease, delay or slowing of disease progression, amelioration or palliation of the disease state, and remission (whether partial or total), whether detectable or undetectable. "Treatment" can also mean prolonging survival as compared to expected survival if not receiving treatment. Those in need of treatment include those already with the condition or disorder as well as those prone to have the condition or disorder or those in which the manifestation of the condition or disorder is to be prevented.

4-HBA as used in accordance with the present invention can in principle be any 4-HBA commercially available. Preferably, it should be at least 90% pure, preferably at least 91% pure, preferably at least 92% pure, preferably at least 93% pure, preferably at least 94% pure, preferably at least 95% pure, preferably at least 96% pure, preferably at least 97% pure, preferably at least 98% pure, preferably at least 99% pure, preferably at least 99.1% pure, preferably at least 99.2% pure, preferably at least 99.3% pure, preferably at least 99.4% pure, preferably at least 99.5% pure, preferably at least 99.6% pure, preferably at least 99.7% pure, preferably at least 99.8% pure, preferably at least 99.9% pure. Furthermore, before use, it should preferably be tested for potential contaminations and only 4-HBA is used in accordance with the present invention which does not contain any potential harmful contaminants. In particular, 4-HBA for use in accordance with the present invention was tested for the presence of residual metals. In one embodiment, 4-HBA for use in accordance with the present invention is produced according to "Good Manufacturing Practice" (GMP) standards.

If reference is made to 4-HBA herein, if not taught otherwise, it includes its derivatives/analogues, and precursors. The terms derivatives and analogues are used herein interchangeably.

4-HBA derivatives and analogues are compounds derived from 4-hydroxybenzoic acid, which is a phenolic acid. The derivatives and analogues include, but are not limited thereto: parabens (ester derivatives) including methylparaben (Methyl 4-hydroxybenzoate), ethylparaben (Ethyl 4-hydroxybenzoate), propylparaben (Propyl 4-hydroxybenzoate), ad butylparaben (Butyl 4-hydroxybenzoate); alkylated derivatives including 4-Alkoxybenzoic acids (e.g., 4-methoxybenzoic acid), 4-Hydroxy-3-methoxybenzoic acid (vanillic acid), and 4-Hydroxy-3,5-dimethoxybenzoic acid (syringic acid); biologically active derivatives including aspirin analogues: substitution of acetyl groups on the hydroxyl group; protocatechuic acid (3,4-Dihydroxybenzoic acid), and gentisic acid (2,5-Dihydroxybenzoic acid); natural analogues including gallic acid (3,4,5-Trihydroxybenzoic acid), and salicylic acid (2-Hydroxybenzoic acid). In a preferred embodiment, the derivatives/analogues are β-resorcylic acid (2,4-dihydroxybenzoic acid), protocatechuic acid (3,4-dihydroxybenzoic acid), and vanillic acid (4-Hydroxy-3-methoxybenzoic acid).

4-HBA is a small molecule, which is derived from L-tyrosine and precursor compounds include phenylalanine, tyrosine, kaempferol, chorismic acid, resveratrol, p-coumaric acid, and also para-aminobenzoic acid (pABA) as well as 4-hydroxyphenylpyruvic acid (4-HPPA), and 4-hydroxybenzaldehyde (4-HBAL).

As mentioned, 4-HBA is used for treatment or prevention of a CoQ₁₀ deficiency in a human subject. CoQ₁₀ deficiency is a disease which is classified as a mitochondrial respiratory chain disorder and is characterized by reduction of CoQ₁₀ levels in tissues or cultured cells associated with biallelic pathogenic variants or heterozygous variants (as for example observed in multisystem atrophy) in one of the genes involved in the biosynthesis of CoQ₁₀. Primary CoQ₁₀ deficiency (also referred to as primary ubiquinone deficiency) is associated with an extremely heterogeneous group of clinical manifestations. The principle clinical manifestations include
1) Central nervous system (CNS) manifestations, which include encephalopathy, seizures, dystonia, developmental delay, spasticity and/or intellectual disability, progressive cerebellar atrophy and ataxia with intellectual disability and seizures, and distal and peripheral motor neuropathy, wherein the age of onset and clinical severity range from fatal neonatal encephalopathy with hypotonia to a late-onset, slowly progressive multiple system atrophy (MSA)-like phenotype, a neurodegenerative disorder characterized by autonomic failure associated with various combinations of parkinsonism, cerebellar ataxia, and pyramidal dysfunction;
2) Renal involvement, which usually manifests as proteinuria in infancy, wherein affected individuals often present initially with Steroid-resistant nephrotic syndrome (SRNS) that leads to end-stage kidney disease (ESKD), followed by an encephalomyopathy with seizures and stroke-like episodes resulting in severe neurologic impairment and ultimately death;
3) Hypertrophic cardiomyopathy (HCM) as well as lactic acidosis followed by a multisystem disease that included HCM;
4) Retinopathy and optic atrophy;
5) Sensorineural hearing loss;
6) Muscle findings including weakness and exercise intolerance.

The kind of clinical manifestations and symptoms also depend on the effected gene. Up to now, eleven genes/enzymes have been identified to be associated with primary CoQ₁₀ deficiency, i.e., PDSS1, PDSS2, HPDL, COQ2, COQ4, COQ5, COQ6, COQ7, COQ8A/ADCK3, COQ8B/ADCK4, and COQ9. Accordingly, in one embodiment, the primary CoQ₁₀ deficiency to be treated in accordance with the present invention is a primary CoQ₁₀ deficiency, preferably caused by defects in PDSS1, PDSS2, HPDL, COQ2, COQ4, COQ5, COQ6, COQ7, COQ8A/ADCK3, COQ8B/ADCK4, or COQ9.

It is also possible that a patient with CoQ₁₀ deficiency has not only one effected gene, but more than one, for example two or three effected genes, preferably two effected genes. For example, a patient may have a defective COQ2 gene and a defective COQ9 gene and thus, might show more or more severe symptoms. Accordingly, in one embodiment, the primary CoQ₁₀ deficiency to be treated in accordance with the present invention is a primary CoQ₁₀ deficiency, preferably caused by defects in PDSS1, PDSS2, HPDL, COQ2, COQ4, COQ5, COQ6, COQ7, COQ8A/ADCK3, COQ8B/ADCK4, or COQ9, or any combination thereof. In another embodiment the primary CoQ₁₀ deficiency is characterized by a combination of defects in COQ2 and COQ9 or COQ7. For the treatment of a CoQ₁₀ deficiency with said combined defects a therapy by use of a combination of 4-HBA and vanillic acid is suitable as defined herein according to the present invention. Other combinations of the aforementioned defects are not defined individually but also encompassed by the present invention, preferably a combination of two.

A summary of the clinical manifestations associated with the corresponding genes is provided in Table 1, derived from Salviati *et al.* 2017 [Updated 2023]. In: Adam et al., editors. GeneReviews® [Internet]. Seattle (WA): University of Washington, Seattle; 1993-2024. Available from: https://www.ncbi.nlm.nih.gov/books/NBK410087/ and modified.

**Table 1: Primary Coenzyme Q₁₀ Deficiency: Genes and Associated Clinical Features**

| **Gene** | **Clinical Features** | | | | | |
|---|---|---|---|---|---|---|
| | Kidney | Heart | Eyes | Haring | Neurological | Muscles |
| *COQ2* | SRNS | HCM | Retinopathy, optic atrophy | SNHL | Encephalopathy², seizures, other³ | Myopathy |
| *COQ4* | | Heart failure, HCM | Retinopathy | SNHL | Encephalopathy², seizures, ataxia, other⁴ | Myopathy |
| *COQ5⁵* | | | Retinopathy | | | |
| *COQ6* | SRNS⁶ | | | | Encephalopathy, seizures | |
| *COQ7* | | | | | Encephalopathy, ID, peripheral neuropathy | Muscle weakness |
| *COQ8A* | | | | | Encephalopathy, cerebellar ataxia (SCAR9) ⁷, dystonia, spasticity, seizures | Exercise intolerance |
| *COQ8B* | SRNS⁶ | | | | ID, seizures | |
| *COQ9* | Tubulopathy | HCM | | | Encephalopathy | Myopathy |
| *PDSS1* | SRNS | | Retinopathy, optic atrophy | | Encephalopathy, peripheral neuropathy, ataxia | |
| *PDSS2* | SRNS | | Retinopathy | | Leigh syndrome, ataxia | |
| *HPDL* | | | | | Seizures, spasticity, developmental delay | |

| | | | | | | |
|---|---|---|---|---|---|---|
| HCM = hypertrophic cardiomyopathy; ID = intellectual disability; SNHL = sensorineural hearing loss; SRNS = steroid-resistant nephrotic syndrome ² Encephalopathy comprises a wide spectrum of brain involvement with different clinical and neuroradiologic features ³ Adult-onset multisystem atrophy-like phenotype ⁴ Severe hypotonia, respiratory insufficiency, cerebellar hypoplasia, slowly progressive neurologic deterioration, spasticity, and intellectual disability ⁵ The link between *COQ5* pathogenic variants and ataxia still needs confirmation. ⁶ Because individuals with *COQ6-* and *COQ8B*-related CoQ₁₀ deficiency were ascertained by the presence of SRNS, the authors cannot exclude the possibility that biallelic pathogenic variants in these two genes could also cause a broader phenotype. *⁷ COQ8A-related* CoQ₁₀ deficiency is also referred to as autosomal recessive spinocerebellar ataxia 9 or SCAR9 (OMIM 612016). | | | | | | |

An extensive overview of the clinical manifestations is also provided in Alcázar-Fabra et al., Free Radical Biology and Medicine 167 (2021), 141-180.

Accordingly, in one embodiment, the treatment in accordance with the present invention is suitable for treating the clinical manifestations and symptoms, respectively, associated with CoQ₁₀ deficiency referred to above.

Furthermore, the CoQ₁₀ deficiency to be treated in accordance with the present invention can not only be a primary CoQ₁₀ deficiency caused by defects of the CoQ₁₀ biosynthesis pathway due to pathological variant enzymes involved in CoQ₁₀ biosynthesis as explained above, but can also be a secondary CoQ₁₀ deficiency. A secondary CoQ₁₀ deficiency can be caused by different factors and diseases, like mitochondrial diseases, medications, such as statins and other drugs that inhibit CoQ₁₀ synthesis, nutritional deficiencies, aging, diabetes, cancer, neurodegenerative disorders (such as Parkinson's), and/or heart failure. Supplementation of the CoQ₁₀ biosynthesis pathway with CoQ₁₀ precursors like 4-HBA and analogs and precursors thereof may also lead to the increased production of CoQ₁₀ counteracting the CoQ₁₀ deficiency. Thus, in one embodiment, the CoQ₁₀ deficiency to be treated in accordance with the present invention is a secondary CoQ₁₀ deficiency, preferably caused by mitochondrial diseases, medications, such as statins and other drugs that inhibit CoQ₁₀ synthesis, nutritional deficiencies, aging, diabetes, cancer, neurodegenerative disorders (such as Parkinson's), and/or heart failure.

The onset of CoQ₁₀ deficiency, in particular of primary CoQ₁₀ deficiency ranges from birth to the seventh decade and the clinical manifestations may differ among the age groups. However, patients of all age groups are amenable for the treatment in accordance with the present invention. The age-of-onset groups may be divided into i) early infancy, which includes children from birth up to about six months of age, meaning that disease manifestation occurs within the first six months of life; ii) early childhood, which includes children from about 6 months of age to about 6 years of age, meaning that clinical manifestation usually occurs between 6 months and 6 years of life, wherein clinical manifestation usually occurs until the 3^{rd} year of life and it is less likely that manifestation occurs afterwards; (iii) adult, which usually includes adults from about 40 to 70 years of age, meaning that disease manifestation occurs at an age ranging from about 40 to 70 years.

COQ2 associated CoQ₁₀ deficiency is associated with the broadest clinical spectrum and age-of-onset among patients. In particular, COQ2 associated CoQ₁₀ deficiency can be divided into three forms: i) a severe early-infantile form, wherein the affected human subject shows clinical symptoms shortly after birth, preferably within the first 6 months after birth, preferably wherein the at least one or more clinical symptoms are selected from the group consisting of: lactic acidosis, kidney dysfunction, cardiomyopathy, epilepsy, and severe encephalopathy; ii) an intermediate form, wherein the affected human subject shows at least one or more clinical symptoms in early childhood, preferably between the age of six months and six years, or within the first 24, 22, 20, 18, 16, 14, 12, 10, 8, or 6 weeks, preferably within the age of 6 months and three years or four years or five years, preferably wherein the at least one or more clinical symptoms are selected from the group consisting of: developmental delay, muscular hypotonia, ataxia, failure to thrive, epilepsy, kidney deterioration with steroid-resistant nephrotic syndrome, and neurodegeneration; and iii) a late-onset form, wherein the affected human subject shows at least one or more clinical symptoms in adult life, preferably between the age of 40 and 70, preferably at an age above 50 years, preferably between the age of 50 and 70, preferably wherein the at least one or more clinical symptoms are selected from the group consisting of: adult-onset dementia, movement abnormalities, visual problems, dysarthria and autonomic dysfunction.

During the study described in Example 1, it has been shown that treatment with 4-HBA resulted in reduction of proteinuria, reduction of lactic acidosis, reduction of vomiting, improvement of muscle strength, improvement of mobility, improvement of exercise tolerance, improvement of motor function, improvement of fine motor function, improvement of alertness, improvement of speech, improvement of social behavior, and gain of weight in the child. These parameters can be measured with standard approaches and methods. For example, the amount of total protein and albumin in a urine sample can be measured by turbidimetric assays, and the lactic acid concentration in the blood by an enzymatic reaction using lactate oxidase or lactate dehydrogenase (LDH). The behavioral and physical parameters (muscle strength, mobility, exercise tolerance, motor function, fine motor function, alertness, speech, social behavior) can be tested by different standardized methods. For example, muscle strength can be tested with manual muscle testing, which evaluates strength through clinician-guided resistance testing, with a handgrip dynameter, or via function tests like timed sit-to-stand tests or lifting tasks. Mobility can for example be tested with a six-minute walk test (6MWT), exercise tolerance can for example be tested with Cardiopulmonary Exercise Testing (CPET), motor function can for example be tested with observations of walking, running, climbing stairs, or standing on one leg or my motor function measures. Fine motoric function can be tested for example by Nine-Hole Peg Test (9HPT) which evaluates manual dexterity by timing how quickly pegs can be placed in holes, or by a finger tapping test. Alternes can be tested for example by a Psychomotor Vigilance Task (PVT) and speech can be tested for example by an automated speech analysis or by a speech therapist for evaluation of articulation, fluency, and phonation. Social behavior can be for example tested by direct observation or video analysis. However, the kind of test also depends on the age group to be tested. The above-referenced tests are especially suitable for adults or children that are able to cooperate during the test phase. For young children, for example until the age of 6 years, the Bayley Scales of Infant and Toddler Development and The Hammersmith Infant Neurological Examination (HINE) are more suitable for testing the treatment efficiency. Otherwise, most tests are not suitable to perform with children in their early childhood and standardization is hardly possible. Accordingly, to determine the treatment effect, usually therapists or physicians observe the behavior of the child and record the improvements and changes over time. The other clinical manifestations referred to above can also be tested by standard test, *e.g.,* the development of retinopathy and optic atrophy can be determined by visual acuity testing, fundoscopy, and/or optical coherence tomography (OCT).

4-HBA and its analogues and precursors cannot only be used for the treatment of CoQ₁₀ deficiency, but also for the prevention, in particular for the prevention of the disease manifestation and the clinical and metabolic symptoms associated therewith. For example, prenatal diagnosis (in particular relevant for the severe early-infancy form) or postnatal diagnosis (in particular relevant for the intermediate and late-onset forms) can be performed. For example, next generation sequencing or real-time PCR can be performed to detect pathogenic variants in the COQ genes. Furthermore, Sanger sequencing can be performed, wherein this method is particular suitable if a gene effect is already known or highly suspected from the family history of the patient. The diagnosis of a primary CoQ₁₀ deficiency is confirmed when biallelic pathogenic variants in any of the COQ genes are found in a patient, and they segregate with the genetic profile of the parents. Identification of a new pathogenic variant needs to be functionally validated to prove the genetic aetiology of the disease.

COQ2 associated CoQ₁₀ deficiency and also CoQ₁₀ deficiencies associated with the other genes referred to above, are not only caused by a specific mutation is the corresponding gene, but different genotypes can lead to CoQ₁₀ deficiency. In case of COQ2 associated CoQ₁₀ deficiency, the protein should at least have residual activity so that the substrate-enhancement therapy with 4-HBA or an analogue or precursor thereof is effective. Known mutations leading to COQ2 associated CoQ₁₀ deficiency are listed in the following Table 2 (derived from Supplementary Table 1 in Herebian et al., Ann Clin Transl Neurol. 4 (2017), 902-908) and are encompassed by the present invention.

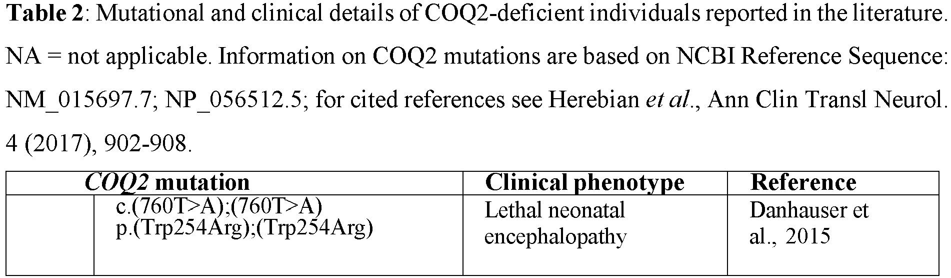

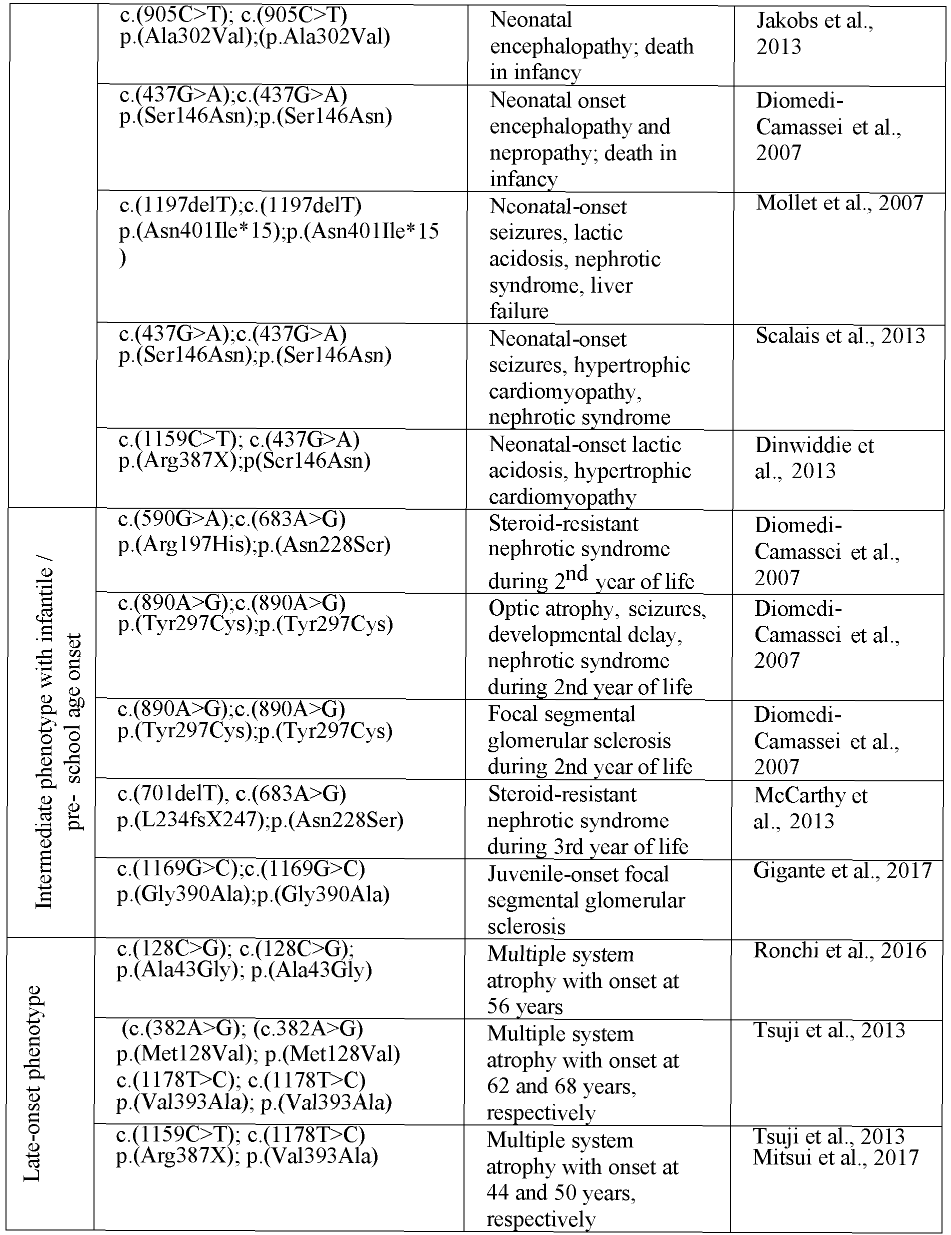

The 4-HBA or an analogue or precursor thereof for use in accordance with the present invention is preferably administered orally with a vehicle to facilitate administration and to dilute the acid. The same applies to embodiments wherein a combination of 4-HBA, the analogue and/or precursor thereof or a composition comprising a combination of 4-HBA, the analogue and/or precursor thereof are preferably administered orally. In general, any vehicle can be used which is suitable for human consumption. In one embodiment, water is used as a vehicle for administration. In another embodiment, milk is used as a vehicle for administration. Milk is particular preferred for administration to children as it masks the taste of 4-HBA. Especially for administration to neonates and infants, breast milk or a breast milk substitute is preferred. Otherwise, cow milk, goat milk, etc. can be used as a vehicle for 4-HBA administration. Preferably, 4-HBA is dissolved in the vehicle before administration. 4-HBA can be provided as loose powder or as encapsulated powder, wherein the latter facilitates dosing. In particular, in one embodiment, 4-HBA is provided as a capsule comprising a defined amount of 4-HBA.

The present invention also relates to a composition comprising 4-HBA or an analogue or precursor thereof or a composition comprising a combination of 4-HBA, the analogue and/or precursor thereof for use in accordance with the present invention. In such a composition 4-HBA or an analogue or precursor thereof is the active ingredient, and the composition can additionally comprise excipients and/or further active ingredients. The composition can be either liquid or dry, preferably as capsule, powder, gel, or liquid.

For capsules, typical excipients include fillers or diluents, such as lactose, microcrystalline cellulose (MCC), starch, and mannitol, which are used to increase the bulk of the formulation. Glidants like colloidal silicon dioxide and talc improve the flowability of the powder during encapsulation, while lubricants such as magnesium stearate and stearic acid reduce friction. Disintegrants, including croscarmellose sodium, sodium starch glycolate, and crospovidone, facilitate capsule dissolution after ingestion. The capsule shell itself is often made of gelatin or hydroxypropyl methylcellulose (HPMC) and may include colorants like titanium dioxide or FD&C dyes to improve appearance.

For powders, commonly used excipients include fillers such as lactose, mannitol, dextrose, or sucrose to provide bulk, and flow aids like colloidal silicon dioxide or talc to ensure smooth handling and packaging. Dispersing agents, such as starch or sodium carboxymethylcellulose, help the powder disperse uniformly when mixed with a liquid. Preservatives like methylparaben or sodium benzoate are included in powders meant for reconstitution to prevent microbial growth. Stabilizers, such as antioxidants (ascorbic acid or tocopherols) or chelating agents (EDTA), help maintain the drug's stability. To improve palatability, flavoring agents like fruit flavors and sweeteners such as saccharin or aspartame may be added. In effervescent powders, citric acid and sodium bicarbonate create the effervescence upon contact with water.

For gels, gelling agents such as carbomers (e.g., Carbopol), hydroxypropyl methylcellulose (HPMC), xanthan gum, or sodium alginate are essential to provide the semi-solid consistency. Solvents like purified water, glycerin, or propylene glycol are used to dissolve or disperse the active ingredient. Stabilizers, including EDTA and antioxidants like tocopherols or butylated hydroxytoluene (BHT), enhance the gel's stability. Preservatives such as parabens or benzalkonium chloride prevent microbial contamination. Humectants like glycerin or sorbitol are added to retain moisture, and surfactants such as polysorbates or lecithin may be included to emulsify hydrophobic ingredients.

For liquids, solvents such as purified water, ethanol, polyethylene glycol (PEG), or propylene glycol are commonly used to dissolve the active ingredient. Sweeteners, including sucrose, sorbitol, or artificial sweeteners like sucralose or aspartame, improve the taste of oral formulations. Viscosity enhancers such as xanthan gum, sodium carboxymethylcellulose, or methylcellulose are used to achieve the desired consistency. For suspensions, suspending agents like microcrystalline cellulose, sodium alginate, or polysorbates help maintain a uniform dispersion of the drug. Preservatives, including parabens, sodium benzoate, or potassium sorbate, ensure microbial safety. Stabilizers like EDTA or antioxidants such as ascorbic acid or tocopherols are included to maintain the chemical integrity of the drug. Flavoring agents, such as essential oils or artificial flavors, and coloring agents like FD&C dyes may also be added to improve the product's acceptability.

Excipients are selected based on the physical and chemical properties of the drug, the requirements of the dosage form, patient preferences, and regulatory compliance. Proper excipient selection is critical to ensure the drug product's stability, effectiveness, and patient acceptability.

In one embodiment, 4-HBA is administered at doses which range from about 50 mg/kg body weight/day to about 250 mg/kg body weight/day, preferably from about 50 mg/kg body weight/day to about 150 mg/kg body weight/day, preferably from about 75 mg/kg body weight/day to about 150 mg/kg body weight/day or from about 70 mg/kg body weight/day to about 140 mg/kg body weight/dy. Preferably, the amount of 4-HBA is administered in three separate doses during the day, preferably wherein the doses are administered six to eight hours apart from each other. In particular, 4-HBA is administered at a dose of about 50 mg/kg body weight/day, 55 mg/kg body weight/day, 60 mg/kg body weight/day, 65 mg/kg body weight/day, 70 mg/kg body weight/day, 75 mg/kg body weight/day, 80 mg/kg body weight/day, 85 mg/kg body weight/day, 90 mg/kg body weight/day, 95 mg/kg body weight/day, 100 mg/kg body weight/day, 105 mg/kg body weight/day, 110 mg/kg body weight/day, 115 mg/kg body weight/day, 120 mg/kg body weight/day, 130 mg/kg body weight/day, 140 mg/kg body weight/day, 150 mg/kg body weight/day, 160 mg/kg body weight/day, 170 mg/kg body weight/day, 180 mg/kg body weight/day, 190 mg/kg body weight/day, 200 mg/kg body weight/day, 210 mg/kg body weight/day, 220 mg/kg body weight/day, 230 mg/kg body weight/day, 240 mg/kg body weight/day, 250 mg/kg body weight/day.

In one embodiment, the 4-HBA dose for administration to children ranges from about 3 x 150 mg/day to about 3 x 850 mg/day, preferably from about 3 x 150 mg/day to about 3 x 500 mg/day, preferably from about 3 x 250 mg/day to about 3 x 500 mg/day. In particular, administration of 4-HBA to a child can be done at a dose of about 3 x 150 mg/day, 3 x 200 mg/day, 3 x 250 mg/day, 3 x 300 mg/day, 3 x 350 mg/day, 3 x 400 mg/day, 3 x 450 mg/day, 3 x 500 mg/day, 3 x 550 mg/day, 3 x 600 mg/day, 3 x 650 mg/day, 3 x 700 mg/day, 3 x 750 mg/day, 3 x 800 mg/day, or 3 x 850 mg/day.

In one embodiment, the 4-HBA dose for administration to adults ranges from about 3 x 250 mg/day to about 3 x 6000 mg/day, preferably from about 3 x 500 mg/day to about 3 x 6000 mg/day, preferably from about 3 x 1000 mg/day to about 3 x 6000 mg/day, preferably from about 3 x 1000 mg/day to about 3 x 3500 mg/day, preferably from about 3 x 1750 mg/day to about 3 x 3500 mg/day. In particular, administration of 4-HBA to an adult can be done at a dose of about 3 x 250 mg/day, 3 x 300 mg/day, 3 x 350 mg/day, 3 x 400 mg/day, 3 x 450 mg/day, 3 x 500 mg/day, 3 x 550 mg/day, 3 x 600 mg/day, 3 x 650 mg/day, 3 x 700 mg/day, 3 x 750 mg/day, 3 x 800 mg/day, 3 x 850 mg/day, 3 x 900 mg/day, 3 x 950 mg/day, 3 x 1000 mg/day, 3 x 1250 mg/day, 3 x 1500 mg/day, 3 x 1750 mg/day, 3 x 2000 mg/day, 3 x 2250 mg/day, 3 x 2500 mg/day, 3 x 2750 mg/day, 3 x 3000 mg/day, 3 x 3250 mg/day, 3 x 3500 mg/day, 3 x 3750 mg/day, 3 x 4000 mg/day, 3 x 4250 mg/day, 3 x 4500 mg/day, 3 x 4750 mg/day, 3 x 5000 mg/day, 3 x 5250 mg/day, 3 x 5500 mg/day, 3 x 5750 mg/day, or 3 x 6000 mg/day.

In one embodiment, the dose is increased over treatment time. In a preferred embodiment, administration is started with a lower dose and within a month, preferably after one week, the dose is increased. In a preferred embodiment, 4-HBA is administered at an initial dose of about 50 mg/kg body weight/day, 55 mg/kg body weight/day, 60 mg/kg body weight/day, 65 mg/kg body weight/day, 70 mg/kg body weight/day, 75 mg/kg body weight/day, 80 mg/kg body weight/day, 85 mg/kg body weight/day, 90 mg/kg body weight/day, 95 mg/kg body weight/day, or 100 mg/kg body weight/day, preferably at a dose of 75 mg/kg body weight/day or 70 mg/kg body weight/day. The subsequent dose is preferably about 100 mg/kg body weight/day, 110 mg/kg body weight/day, 120 mg/kg body weight/day, 130 mg/kg body weight/day, 140 mg/kg body weight/day, 150 mg/kg body weight/day, 160 mg/kg body weight/day, 170 mg/kg body weight/day, 180 mg/kg body weight/day, 190 mg/kg body weight/day, 200 mg/kg body weight/day, 210 mg/kg body weight/day, 220 mg/kg body weight/day, 230 mg/kg body weight/day, 240 mg/kg body weight/day, or 250 mg/kg body weight/day, preferably of 150 mg/kg body weight/day or 140 mg/kg body weight/day within the first month, preferably after the first week of treatment. The first and the subsequent doses are administered daily and preferably in three separate doses as mentioned above. In a preferred embodiment, the initial dose is 75 mg/kg body weight/day and the subsequent dose (until end of treatment) is 150 mg/kg body weight/day. In another preferred embodiment, the initial dose is 70 mg/kg body weight/day and the subsequent dose (until end of treatment) is 140 mg/kg body weight/day. Preferably, the amount of 4-HBA is administered in three separate doses during the day, preferably wherein the doses are administered six to eight hours apart from each other.

In one embodiment, the initial 4-HBA dose for administration to children ranges from about 3 x 150 mg/day to about 3 x 350 mg/day, i.e., about 3 x 150 mg/day, 3 x 160 mg/day, 3 x 170 mg/day, 3 x 180 mg/day, 3 x 190 mg/day, 3 x 200 mg/day, 3 x 210 mg/day, 3 x 220 mg/day, 3 x 230 mg/day, 3 x 240 mg/day, 3 x 250 mg/day, 3 x 260 mg/day, 3 x 270 mg/day, 3 x 280 mg/day, 3 x 290 mg/day, 3 x 300 mg/day, 3 x 310 mg/day, 3 x 320 mg/day, 3 x 330 mg/day, 3 x 340 mg/day, or 3 x 350 mg/day and is preferably about 3 x 250 mg/day, and the subsequent dose ranges from about 3 x 350 mg/day to about 3 x 850 mg/day, i.e., about 3 x 350 mg/day, 3 x 375 mg/day, 3 x 400 mg/day, 3 x 425 mg/day, 3 x 450 mg/day, 3 x 475 mg/day, 500 mg/day, 3 x 525 mg/day, 3 x 550 mg/day, 3 x 575 mg/day, 600 mg/day, 3 x 625 mg/day, 3 x 650 mg/day, 3 x 675 mg/day, 700 mg/day, 3 x 725 mg/day, 3 x 750 mg/day, 3 x 775 mg/day, 800 mg/day, 3 x 825 mg/day, or 3 x 850 mg/day, and is preferably about 3 x 500 mg/day. In another embodiment the dose may be increasing or decreasing over the day e.g. 1 x 450 mg/day, 1 x 450 mg/day and 1 x 500 mg/day or 1 x 570 mg/day, 1 x 500 mg/day and 1 x 450 mg/day.

In one embodiment, the initial 4-HBA dose for administration to adults ranges from about 3 x 250 mg/day to about 3 x 3500 mg/day, preferably from about 3 x 500 mg/day to about 3 x 3500 mg/day, preferably from about 3 x 1000 mg/day to 3 x 3500 mg/day and is preferably 3 x 1750 mg/day, *i.e.,* about 3 x 250 mg/day, 3 x 300 mg/day, 3 x 350 mg/day, 3 x 400 mg/day, 3 x 450 mg/day, 3 x 500 mg/day, 3 x 550 mg/day, 3 x 600 mg/day, 3 x 650 mg/day, 3 x 700 mg/day, 3 x 750 mg/day, 3 x 800 mg/day, 3 x 850 mg/day, 3 x 900 mg/day, 3 x 950 mg/day, 3 x 1000 mg/day, 3 x 1250 mg/day, 3 x 1500 mg/day, 3 x 1750 mg/day, 3 x 2000 mg/day, 3 x 2250 mg/day, 3 x 2500 mg/day, 3 x 2750 mg/day, 3 x 3000 mg/day, 3 x 3250 mg/day, 3 x 3500 mg/day, and the subsequent dose ranges from about 3 x 500 mg/day to about 3 x 6000 mg/day, preferably from about 3 x 750 mg/day to about 3 x 6000 mg/day, preferably form about 3 x 1750 mg/day to about 3 x 6000 mg/day and is preferably about 3 x 3500 mg/day, i.e., about 3 x 500 mg/day, 3 x 550 mg/day, 3 x 600 mg/day, 3 x 650 mg/day, 3 x 700 mg/day, 3 x 750 mg/day, 3 x 800 mg/day, 3 x 850 mg/day, 3 x 900 mg/day, 3 x 950 mg/day, 3 x 1000 mg/day, 3 x 1250 mg/day, 3 x 1500 mg/day, 3 x 1750 mg/day, 3 x 2000 mg/day, 3 x 2250 mg/day, 3 x 2500 mg/day, 3 x 2750 mg/day, 3 x 3000 mg/day, 3 x 3250 mg/day, 3 x 3500 mg/day, 3 x 3750 mg/day, 3 x 4000 mg/day, 3 x 4250 mg/day, 3 x 4500 mg/day, 3 x 4750 mg/day, 3 x 5000 mg/day, 3 x 5250 mg/day, 3 x 5500 mg/day, 3 x 5750 mg/day, or 3 x 6000 mg/day. In another embodiment the dose may be increasing or decreasing over the day e.g. 1 x 1250 mg/day, 1 x 1250 mg/day and 1 x 1500 mg/day or 1 x 1750 mg/day, 1 x 1700 mg/day and 1 x 1650 mg/day.

The present invention also relates to a combination therapy for use in accordance with the present invention. In particular, 4-HBA or an analogue, or precursor thereof can be administered concurrently with or subsequently to CoQ₁₀. Concurrently means in this context, that 4-HBA and CoQ₁₀ can be administered either as one composition or as two separate compositions, wherein both substances are administered at the same time or shortly (within minutes) after each other. Both substances are administered at an effective amount, wherein 4-HBA is preferably administered at the dosages referred to above. CoQ₁₀ is administered at dosages usually used for treatment of CoQ₁₀ deficiency, which is about 30 mg/kg/day. In a preferred embodiment, CoQ₁₀ is orally administered at a dose of about 5 mg/kg body weight per day, 10 mg/kg body weight per day, 15 mg/kg body weight per day, 20 mg/kg body weight per day, 25 mg/kg body weight per day, 30 mg/kg body weight per day, 35 mg/kg body weight per day, 40 mg/kg body weight per day, 45 mg/kg body weight per day, 50 mg/kg body weight per day, 55 mg/kg body weight per day, 60 mg/kg body weight per day, 65 mg/kg body weight per day, or to 70 mg/kg body weight per day, preferably about 20 mg/kg body weight per day to about 40 mg/kg body weight per day, and most preferably at a dose of about 30 mg/kg body weight per day.

Furthermore, the administration of 4-HBA can be combined with the administration of one of its precursors, for example with tyrosine, for example in the form of tyrosine-rich foods or food or dietary supplements comprising tyrosine. Accordingly, in one embodiment, the treatment in accordance with the present invention comprises administration of 4-HBA alone, or in combination with CoQ₁₀, or in combination with one of its precursors, for example tyrosine in the form of tyrosine-rich food or of a food/dietary supplement comprising tyrosine, or with 4-hydroxyphenylpyruvic acid (4-HPPA) or 4-hydroxybenzaldehyde (4-HBAL), or in combination with both, *i.e.,* with CoQ₁₀ and an 4-HBA precursor as referred to above. Especially for mild forms of CoQ₁₀ deficiency, tyrosine in form of tyrosine-rich food or as food supplement can also be used alone for treatment or in combination with CoQ₁₀ at any dosage defined herein.

The subject to be treated can comprise defects in more than one of the enzymes of the CoQ₁₀ biosynthesis pathway. Accordingly, treatment may comprise the simultaneous administration of 4-HBA and of one of its precursors, e.g. vanillic acid to bypass different enzymatic reactions.

Especially for mild forms of CoQ₁₀ deficiency or for treatment of neonates, 4-HBA itself can be used as a food or dietary supplement, preferably in liquid form or powder from, preferably liquid. Accordingly, the present invention also relates to a food or dietary supplement comprising 4-HBA in an amount that is suitable to allow administration of 4-HBA in the above-indicated dosage. A preferred amount is between 100 mg and 500 mg, between 100 mg and 400 mg, between 200 mg and 400 mg, between 200 mg and 300 mg, more preferably 250 mg.

The present invention further relates to a food/dietary supplement comprising a composition comprising at least 4-HBA or an analogue or precursor thereof, preferably wherein the amount of 4-HBA or the analogue or precursor thereof in the composition is between 100 mg to 500 mg, e.g. 100 mg, 125 mg, 150 mg, 175 mg, 200 mg, 225 mg, 250 mg, 275 mg, 300 mg, 325 mg, 350 mg, 375 mg, 400 mg, 425 mg, 450 mg, 475 mg, 500 mg, preferably 250 mg. Preferably, the food/dietary supplement is formulated in a liquid or dry form, preferably as capsule, powder, gel, or liquid. The excipients for preparing the corresponding dosage forms are referred to above. The same applies to another embodiment of a food/dietary supplement comprising a combination of 4-HBA, the analogue and/or precursor thereof or comprising a composition that comprises a combination of 4-HBA, the analogue and/or precursor thereof.

The present invention further relates to a method for identifying and/or obtaining a substance suitable for the amelioration or treatment of CoQ₁₀ deficiency comprising a) contacting a tissue-specific cell model system, preferably neuronal precursor cells (NPCs), differentiated neurons (DNs), or brain organoids with a test substance, wherein the NPCs, DNs and brain organoids have been generated based on iPSCs comprising a pathogenic variant of a protein involved in CoQ₁₀ biosynthesis, and (b) studying the neuronal differentiation in the tissue-specific cell model system, preferably in the NPCs, DNs, or brain organoids, wherein an improved neuronal differentiation in comparison to a control is indicative for a substance suitable for the amelioration or treatment of CoQ₁₀ deficiency.

The present invention further relates to a method for screening a substance for the suitability to ameliorate or treat CoQ₁₀ deficiency comprising (a) contacting a tissue-specific cell model system, preferably neuronal precursor cells (NPCs), differentiated neurons (DNs), or brain organoids with a test substance, wherein the NPCs, DNs and brain organoids have been generated based on iPSCs comprising a pathogenic variant of a protein involved in CoQ₁₀ biosynthesis; (b) studying the neuronal differentiation in the tissue-specific cell model system, preferably in the NPCs, DNs, or brain organoids of step (a); and (c) comparing the neuronal differentiation studied in step (b) to that of the tissue-specific cell model system, preferably NPCs, DNs or brain organoids not subjected to the test substance.

Different parameters, which are known to a person skilled in the art, can be used to study neuronal differentiation. For example, neurite length and number of branch points are measured, and immunofluorescent staining of neuronal markers is performed. These parameters are particularly applied when the assay is performed with DPs. Furthermore, the same approaches, *i.e.,* measurement of neurite length and number of branch points as well as immunofluorescent staining of neuronal markers, and in addition morphological assessment, *e.g.,* measuring the size of the organoid, cytoarchitecture (*e.g.,* organization of neuroepithelial layers), neuronal outgrowth, branching complexity, and synapse formation; functional assays, *e.g.,* electrophysiology (patch clamp) or calcium imaging can be performed, in particular when the assay is performed with brain organoids. Furthermore, multiomix approaches can be applied (*e.g.,* transcriptomics, proteomics, metabolomics).

The present invention further relates to a method for identifying and/or obtaining a substance suitable for the amelioration or treatment of CoQ₁₀ deficiency comprising a) contacting a tissue-specific cell model system, preferably kidney precursor cells (KPCs), podocytes, or kidney organoids with a test substance, wherein the KPCs, podocytes, and kidney organoids have been generated based on iPSCs comprising a pathogenic variant of a protein involved in CoQ₁₀ biosynthesis, and (b) studying the tubular and glomerular structure, function, and integrity in the tissue-specific cell model system, preferably KPCs, podocytes, or kidney organoids, wherein an improved tubular and glomerular structure, function, and integrity in comparison to a control is indicative for a substance suitable for the amelioration or treatment of CoQ₁₀ deficiency.

The present invention further relates to a method for screening a substance for the suitability to ameliorate or treat CoQ₁₀ deficiency comprising (a) contacting a tissue-specific cell model system, preferably kidney precursor cells (KPCs), podocytes, or kidney organoids with a test substance, wherein the KPCs, podocytes, and kidney organoids have been generated based on iPSCs comprising a pathogenic variant of a protein involved in CoQ₁₀ biosynthesis; (b) studying the tubular and glomerular structure, function, and integrity in the tissue-specific cell model system, preferably KPCs, podocytes, or kidney organoids of step (a); and (c) comparing the tubular and glomerular structure, function, and integrity studied in step (b) to that of tissue-specific cell model system, preferably KPCs, podocytes, or kidney organoids not subjected to the test substance.

In an assay with DPs and kidney organoids, the tubular as well as the glomerular structure, function, and integrity in the kidney is assayed according to methods well known to a person skilled in the art as for example described in Jansen et al., Development. 149 (2022), dev200198 and Hale et al., Nat Commun. 9 (2018), 5167. For example, the expression of proteins like nephrin and podocin, of podocyte-specific genes, and/or of organoid-derived glomeruli retain marker can be analyzed as well as the glomerular basement membrane matrisome. Furthermore, structural changes of the organoid can be microscopically assessed and/or gene expression analysis can be performed, for example by single-cell RNA sequencing, super-resolution imaging and electron microscopy. In addition, multiomix approaches can be applied (e.g., transcriptomics, proteomics, metabolomics).

The methods of the present invention are preferably used for screening, identifying and obtaining a substance suitable for the amelioration or treatment of COQ2 associated CoQ₁₀ deficiency or HPDL associated CoQ₁₀ deficiency. In one embodiment, the methods of the present invention are used for screening, identifying and obtaining a 4-HBA analogue/precursor suitable for the treatment of CoQ₁₀ deficiency, preferably of COQ2 associated CoQ₁₀ deficiency or HPDL associated CoQ₁₀ deficiency. In one embodiment, the methods of the present invention are used for screening, identifying and obtaining a 4-HBA analogue/precursor suitable for the treatment of CoQ₁₀ deficiency, preferably of PDSS1, PDSS2, COQ4, COQ5, COQ6, COQ7, COQ8A/ADCK3, COQ8B/ADCK4, and/or COQ9.

The pathogenic variant of the protein involved in CoQ₁₀ biosynthesis can in principle be any pathogenic variant which leads to a reduced functionality of the protein, for example reduced activity or hindered transport, or which leads to an inactivated protein, or which leads to the non-generation of the protein. In case of COQ2, the protein should remain residual activity. In one embodiment, the pathogenic variant of COQ2 is c.779G>A, p.Gly260Glu or (c.571G>C, p.Val191Leu; c.692G>T, p.Ser231Ile), preferably c.779G>A, p.Gly260Glu, or for example any one of the genotypes listed in Table 2. In one embodiment, the pathogenic variant of HPDL is c.779G>A, p.Gly260Glu.

The methods of the present invention are preferably used to screen, identify and/or obtain a drug suitable for the treatment of CoQ₁₀ deficiency.

The present invention further relates to a tissue-specific cell model system comprising induced pluripotent stem cells (iPSC), which comprise a pathogenic variant of a protein involved in CoQ₁₀ biosynthesis. Such tissue-specific cell model system is either a tissue-specific cell model system which comprises differentiated cells, preferably differentiated neurons (DNs) or differentiated podocytes (DPs), or a tissue-specific cell model system which is a human cerebral/brain organoid or a human kidney organoid. In one embodiment, the tissue-specific cell model system of the present invention is generated based on iPSCs comprising a pathogenic variant of one or more proteins, preferably of one or two proteins, more preferably of one protein involved in CoQ₁₀ biosynthesis. These proteins and pathogenic variants of proteins, respectively, involved in CoQ₁₀ biosynthesis are preferably the proteins referred to further above, *i.e.,* PDSS1, PDSS2, HPDL, COQ2, COQ4, COQ5, COQ6, COQ7, COQ8A/ADCK3, COQ8B/ADCK4, and/or COQ9. In a preferred embodiment, the protein is COQ2 or HPLC, most preferably COQ2. The pathogenic variant of the protein involved in CoQ₁₀ biosynthesis can in principle be any pathogenic variant which leads to a reduced functionality of the protein, for example reduced activity or hindered transport, or which leads to an inactivated protein, or which leads to the non-generation of the protein. In case of COQ2, the protein should remain residual activity. In one embodiment, the pathogenic variant of COQ2 is c.779G>A, p.Gly260Glu or (c.571G>C, p.Val191Leu; c.692G>T, p.Ser231Ile), preferably c.779G>A, p.Gly260Glu, or for example any one of the genotypes listed in Table 2. In one embodiment, the pathogenic variant of HPDL is c.779G>A, p.Gly260Glu. Accordingly, the tissue-specific cell model systems of the present invention, in particular the DNs, DPs, and organoids of the present invention, represent a CoQ₁₀ deficiency, preferably a COQ2 associated CoQ₁₀ deficiency or HPDL associated CoQ₁₀ deficiency and are used in one embodiment of the present invention to study the effects of CoQ₁₀ deficiency. Preferably, the tissue-specific cell model systems are used to assess CoQ10 levels, mitochondrial respiration, cellular metabolism and distribution, morphology, protein expression, gene expression, and/or electrophysiology. In one embodiment, the tissue-specific cell model systems are used in the methods of the present invention for screening, identifying and obtaining of a substance suitable for the amelioration or treatment of CoQ₁₀ deficiency. The generation of the tissue-specific cell model system is described in Example 3 in more detail.

Several documents are cited throughout the text of this specification. The contents of all cited references (including literature references, issued patents, published patent applications as cited throughout this application including the background section and manufacturer's specifications, instructions, etc.) are hereby expressly incorporated by reference; however, there is no admission that any document cited is indeed prior art as to the present invention.

A more complete understanding can be obtained by reference to the following specific examples which are provided herein for purposes of illustration only and are not intended to limit the scope of the invention.

Several documents are cited throughout the text of this specification. The contents of all cited references (including literature references, issued patents, published patent applications as cited throughout this application including the background section and manufacturer's specifications, instructions, etc.) are hereby expressly incorporated by reference; however, there is no admission that any document cited is indeed prior art as to the present invention.

A more complete understanding can be obtained by reference to the following specific examples which are provided herein for purposes of illustration only and are not intended to limit the scope of the invention.

### EXAMPLES

### Example 1: Treatment of a child with 4-hydroxybenzoic acid (4-HBA) led to an improvement of metabolic parameters and clinical manifestations of CoQ₁₀ deficiency

In the present study, it was shown for the first time that administration of 4-hydroxybenzoic acid (4-HBA) to a child who was diagnosed with COQ2-associated primary disorder of coenzyme Q₁₀ (CoQ₁₀) biosynthesis and who showed developmental delay and muscular hypotonia, speech delay, chronic lactic acidosis, recurrent vomiting and failure to thrive, generalized edema and a nephrotic syndrome as well as proteinuria and small bilateral lesions in the thalami and a lactate peak in the white matter, led to a remarkable improvement of the metabolic parameters and clinical manifestations of the CoQ₁₀ deficiency.

### Case report (pre-4-HBA treatment)

The boy being subject of the present study is the first child of healthy, non-consanguineous parents with German ancestry. Pregnancy and birth were uneventful. During the first year of life, developmental delay and muscular hypotonia became evident. Unsupported sitting was possible with 12 months; he started to crawl at 17 months. In addition, speech development was delayed. At the age of 2 ½ years the boy was able to speak around 15 single words. Beside the developmental problems, also recurrent vomiting and failure to thrive were noted. Laboratory investigations revealed chronic lactic acidosis. At the age of 2 8/12 years, the boy developed generalized edema and a nephrotic syndrome was diagnosed. Treatment with steroids showed no improvement.

At the age of 3 years, under the suspicion of a metabolic disease, genetic testing (exome sequencing) was applied. Analysis revealed compound-heterozygous variants in the *COQ2* gene (c.571G>C, p.Val191Leu; c.692G>T, p.Ser231Ile). In view of the clinical and genetic data, the diagnosis of a COQ2-associated primary disorder of coenzyme Q₁₀ (CoQ₁₀) biosynthesis was made (primary coenzyme Q₁₀ deficiency-1; COQ10D1; OMIM # 607426.

Based on the diagnosis, an oral supplementation therapy with CoQ₁₀ was started (30 mg/kg body weight per day). Over the time course of three months, the parents noted some improvement in terms of better exercise tolerance (child was more active, less episodes with vomiting and apathy) but no major clinical changes.

### Clinical application of 4-HBA

So far, 4-HBA has not been used as a drug or dietary supplement. However, 4-HBA and its derivatives are applied as supplements in the food and cosmetic industry (E-substances E 214 and E 215). 4-HBA has a GRAS ("Generally Recognized As Safe") status according to the U.S. Food and Drug Administration (FDA).

4-HBA is an acid, which has a potential risk of injuries to the eye, skin and mucosa. However, the pKa value (acid dissociation constant) of 4-HBA is comparable to acetic acid (pKa-values 4.61 vs 4.756) and, for example, the drug acetylsalicylic acid is more acidic compared to 4-HBA (pKa-value 3.48). Accordingly, the application should be safe if the substance is dissolved in milk or drinking water. Apart from extensive safety studies in mice and rats, oral administration of 4-HBA has also been tested in human subjects. Individuals received 5 g 4-HBA every 6 h for 24 h and 26 g 4-HBA in 28 h with drinking water, respectively (GESTIS-Stoffdatenbank: https://gestis.dguv.de/data?name=492602; European Chemicals Agency ECHA: Information on registered substances; BUA Stoffberichte 164-166: Kurzberichte; 164 p-Hydroxybenzoesäure, 12/93; 165. Application of 4-HBA was reported as safe and no side effects occurred.

Treatment of the above-mentioned child with 4-HBA in terms of a compassionate use treatment was considered. For this purpose, 4-HB was obtained with 99.9% purity from BLD Pharmatech GmbH (Reinbek, Germany) and additionally tested for potential contaminations by the company Taros Chemicals GmbH & Co. KG (Dortmund, Germany). The case was discussed and approved by the local ethics board. Subsequently, the 4-HBA was encapsulated at 250 mg doses.

Prior to the start of treatment, laboratory-work up, brain MRI with MR-spectroscopy and a skin biopsy were performed. Laboratory investigations revealed proteinuria (total protein 523 mg/l, norm < 150; albumin 441 mg/l, norm < 30) and lactic acidosis (6.30 mmol/l, norm <1.60). These parameters can be measured by standard laboratory tests, e.g., total protein amount and albumin by turbidimetric assays (see for example Shahangian et al., Am J Clin Pathol. 81 (1984), 651-654, and lactic acid concentration in the blood by an enzymatic reaction using lactate oxidase or lactate dehydrogenase (LDH).

Brain imaging demonstrated small bilateral lesions in the thalami and a lactate peak in the white matter, in line with a mitochondrial disease (Leigh-syndrome pattern). Further work-up (ultrasound of the abdomen, echocardiography, etc.) was normal. The patient was seen by physiotherapists and speech therapists.

For the start of 4-HBA treatment, the oral supplementation with CoQ₁₀ was stopped. Treatment was started orally at a dose of 3 x 250 mg/day (75 mg/kg body weight/day) and after one week increased to 3 x 500 mg/day (150 mg/kg body weight/day). The 4-HBA was dissolved in milk to reduce the acidity. This mode of administration worked fine without clinical problems.

Follow-up over 2 ½ months showed no clinical side effects. Repeated laboratory investigations revealed no abnormalities (e.g. normal blood pictures, electrolytes, liver values, coagulation parameters, etc.). Regarding kidney function, a clear reduction of proteinuria was noted. In particular, after two weeks of 4-HBA treatment, the albumin concentration in the urine decreased from 441 mg/l (pre-treatment) to 86.9 mg/ml and further decreased to 19 mg/l after 8 weeks of 4-HBA treatment. Thus, a normalization of albumin in the urine was already seen after two months of treatment. The total protein concentration in the urine decreased within two weeks of 4-HBA treatment from 523 mg/l (pre-treatment) to 166 mg/l, and further normalized to 54 mg/l after 8 weeks of 4-HBA treatment. Accordingly, the treatment with ramipril was stopped. Metabolic parameters improved as well. In particular, lactate levels decreased from 6.30 mmol/l (pre-treatment) to around 2-3 mmol/l. Clinically, the parents, physiotherapists (exams on a weekly basis), and pediatricians noted a drastic improvement in terms of muscle strengths, mobility, exercise tolerance and fine motor function. The boy is now able to walk unsupported for short distances, which was not possible prior to treatment. He is now able to grab and transfer small objects (marbles) by using a tweezer grip. Also, the eating behavior improved and recurrent vomiting stopped completely. The treatment success can also be seen in the clinical images made at the start of 4-HBA treatment and after 2.5 months of 4-HBA treatment (see Fig. 2). Accordingly, the boy significantly gained weight (1.1 kg in 2 ½ months). Apart from motoric functions, also alertness and speech improved with learning of several new words, which was assessed by speech therapists and pediatricians. Surprisingly, also the social behavior changed. Prior to treatment the child was extremely anxious and not playing age-appropriately. This changed dramatically with much better interaction and participation in supportive treatments. The observations have been made by trained physiotherapists and pediatricians.

### Example 2: Molecular analysis of 4-HBA effects in patient-derived skin fibroblasts

For verification that there is indeed a specific effect of 4-HBA on the endogenous CoQ₁₀ biosynthesis in the child which shows the above-mentioned COQ2 deficiency, a skin biopsy was taken. After culturing of the cells, CoQ₁₀ measurements in 4-HBA treated and untreated cell lines (healthy control fibroblasts and patient-derived fibroblasts) were performed using ultra-performance liquid chromatography coupled to tandem mass spectrometry (UPLC-MS/MS). The detailed method is described in Herebian et al., Ann Clin Transl Neurol. 4 (2017), 902-908. As depicted in Figure 3, the experiments showed a significant improvement of endogenous CoQ₁₀ production after culturing the cells in the presence of 4-HBA.

### Example 3: Production of iPSCs, neural and kidney progenitor cells, differentiated neurons and podocytes as well as brain and kidney organoids and screening methods using the same

Using the approach for generating induced pluripotent stem cells (iPSCs) from human fibroblasts as described in Valente et al., Stem Cell Res 65 (2022), 102971, patient-specific pathogenic variants are introduced into the COQ2 and HPDL genes in iPSCs (a corresponding approach is described in Binder et al., Stem Cell Research 77 (2024), 103395), assuring isogenic control lines. For COQ2, a known pathogenic variant (c.905C>T, p.Ala302Val) is introduced, which causes a severe neonatal-onset phenotype (PMID: 23343605). For HPDL, a known pathogenic variant (c.779G>A, p.Gly260Glu) is introduced, which also causes a severe infantile-onset phenotype (PMID: 32707086). Heterozygous as well as homozygous cell lines are established. Cells are cultured in mTeSR^{™}1 using Matrigel^{®}.

Neural progenitor cells (NPCs) are generated by using the STEMdiff SMADi Neural Induction Kit. For further differentiation into differentiated neurons (DNs), NPCs are cultured in BrainPhys^{™} Neuronal Medium. Generation of brain organoids is carried out according to the detailed protocol described in Le et al., J. Vis. Exp. (2021), e62756, doi: 10.3791/62756. 2D (DNs) and 3D (organoids) cultures can be used to study neuronal differentiation in the COQ2- and HPDL deficient cells. DNs are analyzed at 4 weeks and 8 weeks starting from NPCs (20 biological replicates / 3 independent experiments). In particular, neurite length and number of branch points are measured and immunofluorescent staining of neuronal markers is performed. Brain organoids are analyzed after 40 days and 90 days (10 organoids per line / 3 independent experiments). The neural morphology and physiology on a single cell and network level is checked, including patch-clamp electrophysiology. Subsequently, neuronal morphology with 3D reconstructions of filled neuronal cells is assessed, wherein special emphasis is placed on neuron development and synapse/network maturation. In particular, the size and structure of the organoids are analyzed and transcriptomics, metabolomics and RNA-sequencing are performed. Patch-clamp analysis is performed by measurement of sodium and potassium currents, measurement of repetitive spiking and postsynaptic currents.

Similarly, kidney precursor cells (KPS) and differentiated podocytes (DPs) are prepared based on iPSCs, wherein suitable methods are for example described in Chandrasekaran et al., Sci Rep 11 (2021), 11575 and Bejoy et al. Stem Cell Res Ther 13 (2022), 355. Furthermore, kidney organoids are generated, wherein a detailed protocol for the generation is described in Karp et al., Micromachines (Basel). 13 (2022), 1384. The PDs and kidney organoids are analyzed, wherein in particular in kidney organoids, the glomerular structure, function, and integrity in the kidney is analyzed (see for example Jansen et al., Development. 149 (2022), dev200198 and Hale et al., Nat Commun. 9 (2018), 5167.

The generated DNs, DPs as well as organoids (brain and kidney) are also used in screening methods for the identification of further substances, in particular drugs, that are suitable for the treatment of CoQ₁₀ deficiency. In an assay, the organoids and differentiated cells are exposed to candidate drugs or compounds at varying concentrations for a defined period. Control groups are included, *e.g.,* a negative control (untreated / vehicle-treated organoids/differentiated cells), and/or a positive control (for example a known differentiation enhancer, or organoids/differentiated cells without the gene defect). As a readout for the assay with DPs, neurite length and number of branch points are measured and immunofluorescent staining of neuronal markers is performed. In an assay with brain organoids, neuronal differentiation is determined which can be done for example by morphological assessment, *i.e.,* measuring the size of the organoid, cytoarchitecture (e.g., organization of neuroepithelial layers), neuronal outgrowth, branching complexity, and synapse formation, or by functional assays, *i.e.,* by electrophysiology (patch clamp) or calcium imaging. In addition, multiomix approaches are applied (e.g., transcriptomics, proteomics, metabolomics). In an assay with DPs and kidney organoids, the tubular as well as the glomerular structure, function, and integrity in the kidney is assayed.

## Claims

1. 4-hydroxybenzoic acid (4-HBA), or an analogue or precursor thereof for use in the treatment or prevention of a coenzyme Q₁₀ (CoQ₁₀) deficiency in a human subject.

2. 4-HBA or the analogue or precursor thereof for use according to claim 1, wherein the treatment or prevention is a postnatal treatment or prevention.

3. 4-HBA or the analogue or precursor thereof for use according to claim 1 or 2, wherein the CoQ₁₀ deficiency is primary CoQ₁₀ deficiency.

4. 4-HBA or the analogue or precursor thereof for use according to any one of claims 1 to 3, wherein the CoQ₁₀ deficiency is a 4-HBA-polyprenyltransferase (COQ2)-associated CoQ₁₀ deficiency.

5. 4-HBA or the analogue or precursor thereof for use according to any one of claims 1 to 4, wherein the CoQ₁₀ deficiency is a 4-hydroxyphenylpyruvate dioxygenase-like protein (HPDL)-associated CoQ₁₀ deficiency.

6. 4-HBA or the analogue or precursor thereof for use according to any one of claims 1 to 5, wherein the CoQ₁₀ deficiency is
(i) a severe early-infantile form, wherein the affected human subject shows clinical symptoms shortly after birth, preferably within the first 6 months after birth, preferably wherein the at least one or more clinical symptoms are selected from the group consisting of: lactic acidosis, kidney dysfunction, cardiomyopathy, epilepsy, and severe encephalopathy;
(ii) an intermediate form, wherein the affected human subject shows at least one or more clinical symptoms in early childhood, preferably between the age of six months and six years, preferably wherein the at least one or more clinical symptoms are selected from the group consisting of: developmental delay, muscular hypotonia, ataxia, failure to thrive, epilepsy, kidney deterioration with steroid-resistant nephrotic syndrome, and neurodegeneration; or
(iii) a late-onset form, wherein the affected human subject shows at least one or more clinical symptoms in adult life, preferably wherein the at least one or more clinical symptoms are selected from the group consisting of: adult-onset dementia, movement abnormalities, visual problems, dysarthria and autonomic dysfunction.

7. 4-HBA or the analogue or precursor thereof for use according to any one of claims 1 to 6, wherein the human subject is
(i) a newborn and shows at least one or more of the following clinical symptoms:
lactic acidosis, kidney dysfunction, cardiomyopathy, epilepsy, and severe encephalopathy;
(ii) a child, preferably in its early childhood, and shows at least one or more of the following clinical symptoms: developmental delay, muscular hypotonia, ataxia, epilepsy, failure to thrive, kidney deterioration with steroid-resistant nephrotic syndrome, and neurodegeneration; or
(iii) an adult and shows at least one or more of the following clinical symptoms:
adult-onset dementia, movement abnormalities, visual problems, dysarthria and autonomic dysfunction.

8. 4-HBA or the analogue or precursor thereof for use according to any one of claims 1 to 7, wherein the human subject is a child, preferably in its early childhood, and
(i) has proteinuria as measured by the total protein level and albumin level in a urine sample, wherein the total protein level and albumin level are above the reference range, preferably wherein the total protein level is > 150 mg/ml and the albumin level is > 30 mg/ml in the urine sample,
(ii) has lactic acidosis as measured by the lactate level in a blood sample, wherein the lactate level is above the reference range, preferably wherein the lactate level in the blood sample is >1.6 mmol/l,
(iii) has brain abnormalities including white matter changes, symmetrical lesions of basal ganglia and/or brain stem, cerebellar hypoplasia or atrophy, and a lactate peak detected by brain imaging;
(iv) shows gastrointestinal symptoms including recurrent vomiting and feeding problems
(v) shows reduced muscle strength, mobility, exercise tolerance, motor function, and/or fine motor function compared to peers;
(vi) shows reduced alertness, poorer speech and/or poorer social behavior compared to peers; and/or
(vii) has reduced body weight compared to peers.

9. 4-HBA or the analogue or precursor thereof for use according to any one of claims 1 to 8, wherein 4-HBA or the analogue or precursor thereof is orally administered at a dose of
(i) 50 mg/kg body weight/day to 250 mg/kg body weight/day, preferably of 75 mg/kg body weight/day to 150 mg/kg body weight/day or 70 mg/kg body weight/day to 140 mg/kg body weight/day, preferably wherein the dose is administered in three separate doses approximately 6 to 8 hours apart; or
(ii) at an initial dose of 50 mg/kg body weight/day to 100 mg/kg body weight/day, preferably at a dose of 75 mg/kg body weight/day or 70 mg/kg body weight/day, followed by a subsequent dose of 100 mg/kg body weight/day to 250 mg/kg body weight/day, preferably of 150 mg/kg body weight/day or 140 mg/kg body weight/day, wherein the switch from the initial dose to the subsequent dose is made within a month, preferably after one week of treatment, and preferably wherein the dose is administered every day in three separate doses approximately 6 to 8 hours apart.

10. 4-HBA or the analogue or precursor thereof for use according to any one of claims 1 to 9, wherein the human subject is treated with 4-HBA or an analogue or precursor thereof alone or in combination with CoQ₁₀, preferably wherein 4-HBA or the analogue or precursor thereof is administered concurrently with or subsequently to CoQ₁₀, preferably wherein 4-HBA or the analogue or precursor thereof is administered subsequently to CoQ₁₀, preferably wherein CoQ₁₀ is orally administered at a dose of about 5 mg/kg body weight per day to about 70 mg/kg body weight per day, preferably about 20 mg/kg body weight per day to about 40 mg/kg body weight per day, preferably at a dose of about 30 mg/kg body weight per day.

11. A composition comprising 4-HBA or the analogue or precursor thereof for use according to any one of claims 1 to 10.

12. 4-HBA or an analogue or precursor thereof for use as a drug or use of 4-HBA or an analogue or precursor thereof as a component of a food/dietary supplement.

13. Food/dietary supplement comprising a composition comprising at least 4-HBA or an analogue or precursor thereof, preferably wherein the amount of 4-HBA or the analogue or precursor thereof in the composition is 100 mg to 500 mg, preferably 250 mg.

14. A method for identifying and/or obtaining a substance suitable for the amelioration or treatment of CoQ₁₀ deficiency comprising
(a) contacting neuronal precursor cells (NPCs), differentiated neurons (DNs), or brain organoids with a test substance, wherein the NPCs, DNs and brain organoids have been generated based on iPSCs comprising a pathogenic variant of a protein involved in CoQ₁₀ biosynthesis, and
(b) studying the neuronal differentiation in the NPCs, DNs, or brain organoids, wherein an improved neuronal differentiation in comparison to a control is indicative for a substance suitable for the amelioration or treatment of CoQ₁₀ deficiency; or
(A) contacting kidney precursor cells (KPCs), podocytes, or kidney organoids with a test substance, wherein the KPCs, podocytes, and kidney organoids have been generated based on iPSCs comprising a pathogenic variant of a protein involved in CoQ₁₀ biosynthesis, and
(B) studying the tubular and glomerular structure, function, and integrity in the KPCs, podocytes, or kidney organoids, wherein an improved tubular and glomerular structure, function, and integrity in comparison to a control is indicative for a substance suitable for the amelioration or treatment of CoQ₁₀ deficiency,
preferably wherein the protein involved in CoQ₁₀ biosynthesis is COQ2 or HPDL, and wherein the CoQ₁₀ deficiency is COQ2 associated CoQ₁₀ deficiency or HPLD associated CoQ₁₀ deficiency.

15. A method for screening a substance for the suitability to ameliorate or treat CoQ₁₀ deficiency comprising
(a) contacting neuronal precursor cells (NPCs), differentiated neurons (DNs), or brain organoids with a test substance, wherein the NPCs, DNs and brain organoids have been generated based on iPSCs comprising a pathogenic variant of a protein involved in CoQ₁₀ biosynthesis;
(b) studying the neuronal differentiation in the NPCs, DNs, or brain organoids of step (a); and
(c) comparing the neuronal differentiation studied in step (b) to that of DNs or brain organoids not subjected to the test substance; or
(A) contacting kidney precursor cells (KPCs), podocytes, or kidney organoids with a test substance, wherein the KPCs, podocytes, and kidney organoids have been generated based on iPSCs comprising a pathogenic variant of a protein involved in CoQ₁₀ biosynthesis;
(B) studying the tubular and glomerular structure, function, and integrity in the KPCs, podocytes, or kidney organoids of step (A); and
(C) comparing the tubular and glomerular structure, function, and integrity studied in step (B) to that of KPCs, podocytes, or kidney organoids not subjected to the test substance,
preferably wherein the protein involved in CoQ₁₀ biosynthesis is COQ2 or HPDL, and wherein the CoQ₁₀ deficiency is COQ2 associated CoQ₁₀ deficiency or HPLD associated CoQ₁₀ deficiency.
